(19) Europäisches Patentamt
European Patent Office
Office européen des brevets

(11) Publication number: **0 276 823 B1**

(12) # EUROPEAN PATENT SPECIFICATION

(45) Date of publication of patent specification: **23.09.92**

(51) Int. Cl.⁵: **C07J 9/00**, C07J 41/00, A61K 31/575, C07J 71/00

(21) Application number: **88101139.9**

(22) Date of filing: **27.01.88**

(54) **14,15-Substituted lanosterols and their use as hypocholesterolemic agents.**

(30) Priority: **30.01.87 US 8812**
**28.08.87 US 90634**

(43) Date of publication of application:
**03.08.88 Bulletin 88/31**

(45) Publication of the grant of the patent:
**23.09.92 Bulletin 92/39**

(84) Designated Contracting States:
**AT BE CH DE ES FR GB GR IT LI LU NL SE**

(56) References cited:
**US-A- 4 202 891**

**TETRAHEDRON LETTERS, no. 15, 1966, pages 1677-1680, Pergamon Press Ltd, Oxford, GB; J. FRIED et al.: "32-hydroxylated lanostane derivatives by photolysis of 3beta-Acetoxy-7-ketolanostane"**

**TETRAHEDRON LETTERS, no. 29, 1965, pages 2497-2498, Pergamon Press Ltd, Oxford, GB; T.J. BENTLEY et al.: "The synthesis of 32-oxygenated lanostane derivatives"**

(73) Proprietor: **THE DU PONT MERCK PHARMACEUTICAL COMPANY**
**Barley Mill Plaza, Building 25**
**Wilmington, Delaware 19880-0025(US)**

(72) Inventor: **Gaylor, James Leroy**
**18 Kent Drive**
**Hockessin Delaware 19707(US)**
Inventor: **Johnson, Robert Paul**
**377 Hobart Drive**
**Newark Delaware 19713(US)**
Inventor: **Ko, Soo Sung**
**3215 Drexel Drive**
**Wilmington Delaware 19810(US)**
Inventor: **Magolda, Ronald Louis**
**239 Bishop Drive**
**Aston Pennsylvania 19014(US)**
Inventor: **Stam, Simon Hendrik**
**36 Chatham Court**
**San Jose California 95139(US)**
Inventor: **Trzaskos, James Michael**
**3666 Marian Drive Woodland Acres**
**Boothwyn Pennsylvania 19061(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

CHEMICAL AND PHARMACEUTICAL BULLETIN, vol. 35, no. 1, January 1987, pages 394-397, Tokyo, JP; Y. SONODA et al.: "A simplified synthesis of 32-oxygenated lanosterol derivatives"

CHEMICAL ABSTRACTS, vol. 104, no. 17, 28th April 1986, page 250, no. 144071n, Columbus, Ohio, US; M. MASUO et al.: "Inhibitory effect of 15-oxygenated sterols on cholesterol synthesis from 24,25-dihydrolanosterol", & J. BIOCHEM. (TOKYO) 1986, 99(2), 597-600

CHEMICAL ABSTRACTS, vol. 95, no. 17, 26th October 1981, page 450, no. 147975a, Columbus, Ohio, US; C. TABACIK et al.: "Post-HMG CoA reductase regulation of cholesterol biosynthesis in normal human lymphocytes: lanosten-3beta-ol-32-al, a natural inhibitor"

JOURNAL OF THE CHEMICAL SOCIETY, CHEMICAL COMMUNICATIONS, 1974, pages 477-478, London, GB; T.E. SPIKE et al.: "Structure of a potential intermediate in cholesterol biosynthesis"

TETRAHEDRON LETTERS, no. 49, 1976, pages 4401-4404, Pergamon Press, Oxford, GB; E.J. PARISH et al.: "Synthesis and structure of 15-oxygenated 5alpha, 14beta-cholest-7-en-3beta-ol derivatives"

THE JOURNAL OF BIOLOGICAL CHEMISTRY, vol. 262, no. 3, 25th January 1987, pages 1239-1243, The American Society of Biological Chemists, Inc., Baltimore, US; Y. AOYAMA et al.: "Metabolism of 32-hydroxy-24,25-dihydrolanosterol by purified cytochrome P-45014dm from yeast"

CHEMICAL ABSTRACTS, vol. 92, no. 17, 28th April 1980, page 350, no. 144006d, Columbus, Ohio, US; G.F. GIBBONS et al.: "Regulation of cholesterol biosynthesis in cultured cells by probable natural precursor sterols", & J. BIOL. CHEM. 1980, 255(2), 395-400

JOURNAL OF THE CHEMICAL SOCIETY PERKIN TRANSACTIONS I, 1972, pages 739-748, London, GB; P.L. BATTEN et al.: "The synthesis of some 32-functionalised lanostane derivatives"

(74) Representative: **von Kreisler, Alek et al**
**Patentanwälte Von Kreisler-Schönwald-Fues-Keller Selting-Werner**
**Deichmannhaus am Hauptbahnhof**
**W-5000 Köln 1(DE)**

JOURNAL OF THE CHEMICAL SOCIETY, CHEMICAL COMMUNICATIONS, 1975, pages 213-214, London, GB; G.F. GIBBONS et al.: "Synthesis and configuration at C-15 of the epimeric 5alpha-lanost-8-en-3beta,15-diols"

## Description

The present invention relates to 14,15-substituted lanosterols and to pharmaceutical compositions containing such compounds. These compounds inhibit the activity of lanosta-8,24-dien-3β-ol 14α-methyl-demethylase and suppress the activity 3-hydroxy-3-methylglutaryl coenzyme A reductase (HMGR), two enzymes which are important in cholesterol biosynthesis. The overall effect of these 14,15 substituted lanosterols is to decrease cholesterol formation, thereby resulting in lower serum cholesterol levels in mammals.

Elevated concentrations of serum cholesterol have been demonstrated by a number of clinical studies to be a major contributing factor in the development and progression of atherosclerosis, a disease characterized by the formation of cholesterol-containing plaques in the aorta and lesser arteries. The plaques tend to clog the arterial passage ways, making it difficult, if not impossible, for blood to flow from the heart to various tissues in the body. This pathobiological condition can ultimately lead to Coronary Heart Disease (CDH). See, e.g., Kannel et al. Ann. Intern. Med., 90:85-91 (1979); Final Report of the Pooling Project, J. Chron. Dis., 31:201-306 (1978). By maintaining low cholesterol levels in the blood, arterial plaque formation and CHD can potentially be avoided. See, e.g., Brensike et al., Circulation, 69:313-324 (1984) and Levy et al. Circulation, 69:325-337 (1984).

In mammals, serum cholesterol is derived from exogenous dietary sources as well as through endogenous de novo synthesis. Endogenous synthesis of cholesterol involves a complex set of enzyme-catalyzed reactions and regulatory mechanisms which to date are only partially understood. As Rodwell et al., Adv. Lipid Res., 14:1-74 (1976) indicate, 3-hydroxy-3-methylglutaryl coenzyme A reductase (HMGR) is generally accepted as the rate-limiting enzyme which controls cholesterol biosynthesis from acetyl-CoA in all organisms. Brown et al., J. Lipid Res., 21:505-517 (1980) have shown that regulation of HMGR is a complex process which is under a feedback control mechanism involving both steroidal as well as nonsteroidal isoprenoid metabolites. The authors point out that under normal conditions, the ability of cholesterol to regulate its own biosynthesis when associated with lipoprotein particles is one aspect of this feedback control mechanism. In addition, it has been demonstrated that various oxygenated sterols, when used in a highly purified state, are even more effective than cholesterol in attenuating the amount of HMGR activity, see Breslow et al., Biochem. Biophys. Acta, 398:10-17 (1975), Kandutsch et al., J. Biol. Chem., 252:409-415 (1977), and Chen et al., J. Biol. Chem., 254:715-720 (1979), leading to the hypothesis that oxysterols may also be endogenous mediators which regulate HMGR activity and cholesterol synthesis in situ. See Kandutsch et al., Science, 201:498-501 (1978).

This proposition stimulated considerable research activity. See, e.g., Chen et al., J. Biol. Chem., 254: 715-720 (1979); Havel et al., J. Biol. Chem., 254:9573-9582 (1979); Chang et al., J. Biol. Chem., 255:7787-7795 (1980); Chorvat, U.S. Patent 4,230,626 (1980); Gibbons et al., J. Biol. Chem., 255: 395-400, (1980); Kandutsch et al., J. Biol. Chem., 255:10814- 10821 (1980); Cavenee et al., J. Biol. Chem., 256:2675- 2681 (1981); Tanaka et al., J. Biol. Chem., 258: 13331-13339 (1983); and Trzaskos et al., Fed. Proc., 44:656, (1985). As a result, a number of inhibitors of HMGR activity have been found.

Gibbons et al., J. Biol. Chem., 255:395-400 (1980), for example, have shown that certain synthetic oxygenated lanosterol derivatives are active inhibitors of HMGR activity. Trzaskos et al., Fed. Proc., 44:656 (1985) have established that in situ generation of the Gibbons compounds leads to attenuated HMGR activity and decreased cholesterol biosynthesis.

In addition, Schroepfer et al., U. S. Patent 4,202,891 and Schroepfer et al., Proc. Natl. Acad. Sci. USA, 81:6861-6865 (1984) have revealed that other oxygenated lanosterol derivatives may be successfully employed to lower serum cholesterol levels in animals.

US-A-4 202 891 relates to 15-oxygenated sterols, having the ability to inhibit the biosynthesis of mevalonic acid. The substituent at the 14-position in the compounds disclosed therein is hydrogen or a $C_1$-$C_6$ alkyl group.

In Tetrahedron Letters, 49, 4401 (1976) by E.J. Parrish a number of 15-oxygenated sterols are disclosed. In detail, it is disclosed therein a 5α,14β-cholest-7-en-3β,15β-diol.

In J. Chem. Soc., Perkin I, 739 (1972) by P.L. Batten it is disclosed that 32-oxygenated derivatives of lanosterol are intermediates in the biosynthesis of cholesterol. In detail, 3β-acetoxy-32-acetylaminolanost-7-ene is disclosed.

Chemical Abstracts 104: 144071n (1986) discloses also the δ8-epimer as well as the O-acetyl derivatives of 15-oxygenated sterols.

Chemical Abstracts 95: 147975s (1981) discloses lanosten-3β-ol-32-al.

Additional compounds which affect HMGR and/or other enzymes critical to serum cholesterol biosynthesis are needed. The present invention is directed to this end.

The present invention provides novel 14,15-substituted lanosterol compounds of the formula:

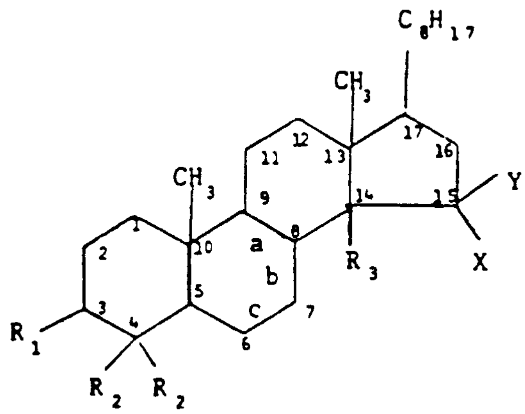

[I]

in which formula the ring structure may be fully saturated or may be unsaturated between one of carbon positions 6-7, 7-8 or 8-9 or between both carbon positions 6-7 and 8-9; and wherein

| | |
|---|---|
| $R_1$ | is =O, $OW_1$, or $OCOW_1$; |
| $R_2$ | is H, $C_1$-$C_6$ alkyl, $C_2$-$C_6$ alkenyl, $C_2$-$C_6$ alkynyl or aryl-$C_1$-$C_6$-alkyl; |
| $R_3$ | is $C_2$-$C_6$ alkenyl, $C_2$-$C_6$ alkynyl, aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$; |
| $R_4$ | is H, $C_1$-$C_6$ alkyl, $C_2$-$C_6$ alkenyl, aryl, aryl-$C_1$-$C_6$-alkyl, or $C_2$-$C_6$ alkynyl; |
| $R_5$ | is $COW_3$, $CSW_3$ or $C(NR_4)W_3$; |
| X and Y, | independently, are H, $C_1$-$C_6$ alkyl, Z, $OR_4$, $OR_5$, $SR_4$, $SR_5$, $N(R_4)_2$, $N(R_5)_2$, $NR_4R_5$, $NR_4OR_4$ or $NR_4OR_5$, |
| | or |
| X and Y, | taken together, are $NR_4$, $NR_5$, $NOR_4$, $NOR_5$, S, $C(R_4)_2$, $C(R_5)_2$, $CR_5R_4$, or O; |
| Z | is halogen; |
| $W_1$ | is H, $C_1$-$C_{20}$ alkyl, $C_2$-$C_{20}$ alkenyl, aryl, aryl-$C_1$-$C_{20}$-alkyl, or $C_2$-$C_{20}$ alkynyl, |
| $W_3$ | is H, $C_1$-$C_6$ alkyl, $C_2$-$C_6$ alkenyl, aryl, aryl-$C_1$-$C_6$-alkyl, $C_2$-$C_6$ alkynyl, $OR_4$ or $N(R_4)_2$; |

and their physiologically acceptable salts;
provided that when X is OH, $OR_5$, $N(R_4)_2$, $N(R_5)_2$, $NR_4R_5$, $SR_5$, $NR_4OR_4$, or $NR_4OR_5$, then Y is other than Z, OH, $OR_5$, $SR_5$, $NR_4OR_4$, $NR_4OR_5$, $N(R_4)_2$, or $N(R_5)_2$.

The above compounds are effective inhibitors of lanosta-8,24-dien-3$\beta$-ol 14$\alpha$-methyl-demethylase activity and suppressants of 3-hydroxy-3-methylglutaryl coenzyme A reductase (HMGR) activity. By interfering with these enzymes, both of which are essential in the cholesterol biosynthetic pathway, cholesterol formation is decreased and serum cholesterol levels lowered.

Thus, the present invention also includes therapeutic pharmaceutical compositions for inhibiting lanosta-8,24-dien-3$\beta$-ol 14$\alpha$-methyl-demethylase activity, suppressing HMGR activity, decreasing cholesterol formation and lowering serum cholesterol levels in mammals. The pharmaceutical compositions comprise (i) an effective amount of a compound of the formula:

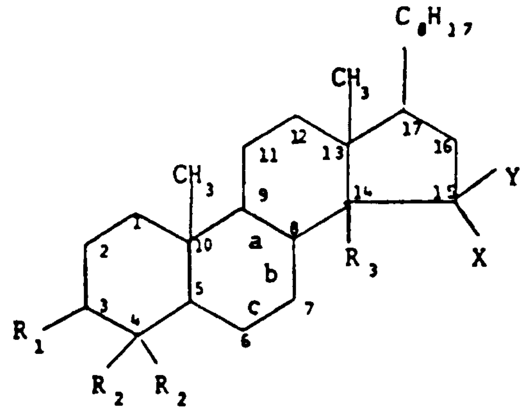

[I]

in which formula the ring structure may be fully saturated or may be unsaturated between one of carbon positions 6-7, 7-8 or 8-9 or between both carbon positions 6-7 and 8-9; and wherein

| | |
|---|---|
| $R_1$ | is =O, $OW_1$, or $OCOW_1$; |
| $R_2$ | is H, $C_1$-$C_6$ alkyl, $C_2$-$C_6$ alkenyl, $C_2$-$C_6$ alkynyl or aryl-$C_1$-$C_6$-alkyl; |
| $R_3$ | is $C_2$-$C_6$ alkenyl, $C_2$-$C_6$ alkynyl, aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$; |
| $R_4$ | is H, $C_1$-$C_6$ alkyl, $C_2$-$C_6$ alkenyl, aryl, aryl-$C_1$-$C_6$-alkyl, or $C_2$-$C_6$ alkynyl; |
| $R_5$ | is $COW_3$, $CSW_3$, or $C(NR_4)W_3$; |
| X and Y, | independently, are H, $C_1$-$C_6$ alkyl, Z, $OR_4$, $OR_5$, $SR_4SR_5$, $N(R_4)_2$, $N(R_5)_2$, $NR_4R_5$, $NR_4OR_4$, $NR_4OR_5$; |
| | or |
| X and Y, | taken together, are $NR_4$, $NR_5$, $NOR_4$, $NOR_5$, S, $C(R_4)_2$, $C(R_5)_2$, $CR_5R_4$, or O; |
| Z | is halogen; |
| $W_1$ | is H, $C_1$-$C_{20}$ alkyl, $C_2$-$C_{20}$ alkenyl, aryl, aryl-$C_1$-$C_{20}$-alkyl, or $C_2$-$C_{20}$ alkynyl; |
| $W_3$ | is H, $C_1$-$C_6$ alkyl, $C_2$-$C_6$ alkenyl, aryl, aryl-$C_1$-$C_6$-alkyl, $C_2$-$C_6$ alkynyl, $OR_4$, or $N(R_4)_2$; |

and their physiologically acceptable salts
provided that when X is OH, $OR_5$, $N(R_4)_2$, $N(R_5)_2$, $NR_4R_5$, $SR_5$, $NR_4OR_4$, or $NR_4OR_5$, then Y is other than Z, OH, $OR_5$, $SR_5$, $NR_4OR_4$, $NR_4OR_5$, $N(R_4)_2$, or $n(R_5)_2$;
and
(ii) a pharmaceutically acceptable carrier or diluent.

In the above formulas, the ring structure may be fully saturated, or may be unsaturated between one of carbon positions 6-7, 7-8 or 8-9 or between both carbon positions 6-7 and 8-9. For convenience, the compounds are designated herein as the "d" compound when fully saturated, the "a" compound when unsaturated in the 8-9 position, the "b" compound when unsaturated in the 7-8 postion, the "c" compound when unsaturated in the 6-7 position, and the "a/c" compound when unsaturated in both positions 6-7 and 8-9.

As used herein, the substituent designated as "poly-($OR_4$, $OR_5$, expoxy) $C_1$-$C_6$ alkyl" shall be taken to mean a $C_1$ to $C_6$ alkyl chain substituted with one or more of any combination of $OR_4$, $OR_5$ and epoxy.

As used herein, the term "alkyl", employed either alone or in combination with other terms such as "poly-($OR_4$ , $OR_5$, expoxy) $C_1$-$C_6$ alkyl" or "arylalkyl", denotes straight chain or branched alkyl, e.g., methyl, ethyl, n-propyl, isopropyl, and the different butyl, pentyl or hexyl isomers.

As used herein, the term "alkenyl", employed either alone or in combination with other terms, denotes straight chain or branched mono- or poly-unsaturated alkyl, e.g., vinyl, propenyl (allyl), crotyl, isopentenyl, and different butenyl, pentenyl, hexadienyl and hexenyl isomers.

As used herein, the term "alkynyl", employed either alone or in combination with other terms, denotes straight chain or branched mono- or poly-unsaturated alkyl, e.g., ethynyl, propynyl (propargyl), 2-butynyl and other butynyl isomers, and the different pentynyl, hexadiynyl and hexynyl isomers.

As used herein, the term "acyl", employed either alone or in combination with other terms, denotes a carbonyl group attached to an alkyl, alkenyl, alkynyl, arylalkyl or aryl group e.g. acetate, butyrate, benzoate, and different alkyl, alkenyl, alkynyl, or aryl isomers.

As used herein, the term "halogen" denotes fluorine, chlorine, bromine and iodine.

With respect to the above compound, composition, and method of use formulas, preferred categories of compounds are:

1. Compounds wherein
   $R_2$ is H, $C_1$-$C_6$ alkyl, or aryl-$C_1$-$C_6$-alkyl;
   $R_3$ is $C_2$-$C_6$ alkenyl, $C_2$-$C_6$ alkynyl, aryl-$C_1$-$C_6$-alkyl, and
   X and Y, independently, are H, $C_1$-$C_6$ alkyl, Z, $OR_4$, $OR_5$, $SR_4$, $SR_5$, $N(R_4)_2$, $N(R_5)_2$ $NR_4R_5$, $NR_4OR_4$, or $NR_4OR_5$;
   or X and Y, taken together, are $NR_4$, $NR_5$, $NOR_4$, $NOR_5$, S, $C(R_4)_2$, $C(R_3)_2$, or $CR_5R_4$.
2. Compounds wherein
   $R_2$ is $C_2$-$C_6$ alkenyl, or $C_2$-$C_6$ alkynl.
3. Compounds wherein
   $R_3$ is $CR_4=CR_4C(R_4)_2Z$, $CR_4=CR_4C(R_4)_2OR_5$,$CR_4=CR_4C(R_4)_2OR_4$.
4. Compounds wherein
   X and Y, taken together, are O.
5. Compounds wherein
   $R_3$ is $C_2$-$C_6$ alkenyl, aryl-$C_1$-$C_6$-alkyl, $C_2$-$C_6$ alkynyl.
6. Compounds wherein
   $R_3$ is $C_2$-$C_6$ alkenyl, aryl-$C_1$-$C_6$-alkyl,
7. Compounds wherein
   X and Y, independently, are H, $C_1$-$C_6$ alkyl, $OR_4$, $OR_5$, $SR_5$, $SR_4$, $N(R_4)_3$, $N(R_5)_2$, $NR_4R_5$, $NR_4OR_4$, or $NR_4OR_5$;
   or X and Y, taken together, are $NR_4$, $NR_5$, $NOR_4$, $NOR_5$, S, $C(R_4)_2$, $C(R_5)_2$, or $CR_4R_5$.
8. Compounds wherein
   X and Y, independently, are H, $C_1$-$C_6$ alkyl, Z, $OR_4$, $OR_5$, $SR_5$, $SR_4$, $N(R_4)_2$, $NR_4OR_4$, $NR_4OR_5$, $N(R_5)_2$ or $NR_4R_5$; and
   or X and Y, taken together, are $NR_4$, $NR_5$, $NOR_5$, S, $C(R_4)_2$, $C(R_5)_2$, or $CR_4R_5$.
9. Compounds wherein
   $R_1$ is OH;
   $R_2$ is H, or $CH_3$;
   $R_3$ is $CH_2CH=CH_2$ and
   X and Y, independently, are H, F, or OH;
   or X and Y, taken together, are NOH.
10. Compounds wherein
   $R_1$ is OH;
   $R_2$ is H, or $CH_3$;
   $R_3$ is $CH_2CH=CH_2$; and
   X and Y, independently, are H or OH.

Many of the above compounds are preferable for reasons of increased ease of synthesis and/or greater efficacy.

Specifically preferred for reasons of greatest ease of synthesis and/or greatest efficacy are:

- 4,4-dimethyl-14α-(1'-hydroxy-2'-propenyl)-5α-cholest-8-en-3β-ol;
- 14α-allyl-4,4-dimethyl-5α-cholest-8-en-3β-ol-15-oxime;

The compounds of the above formulas can be employed to inhibit lanosta-8,24-dien-3β-ol 14α-methyl-demethylase activity, suppress HMGR activity, decrease cholesterol formation and lower serum cholesterol levels in mammals. These compounds can be administered alone, or in combination with pharmaceutically acceptable carriers or diluents appropriate to the indicated route of administration. Administration can be oral, sublingual, buccal, topical and parenteral such as intravenous, subcutaneous or intramuscular. Acceptable carriers and diluents are well-known in the pharmaceutical art, and are described, for example, in Remington's Pharmaceutical Sciences, Gennaro, A. R., ed., Mack Publishing Co., Easton, PA (1985). The useful dosage to be administered and the mode of administration will vary depending upon the age, weight and species of mammal treated.

Briefly, the mechanism by which the active 14,15-substituted lanosterol compounds of the present invention are believed to function is as follows. First, the observed decrease in HMGR activity is thought to occur as a result of a decreased synthesis of HMGR protein and/or an enhanced rate of HMGR degradation (collectively termed herein as "suppression"). The observed decrease in lanosta-8,24-dien-3β-ol 14α-methyl demethylase activity is thought to occur as a result of a direct action of the compounds on the demethylase enzyme (termed herein as "inhibition"). The inhibition of lanosta-8,24-dien-3β-ol 14α-methyl demethylase

activity is also thought to result in the production of molecules which in turn act as suppressors of HMGR activity as described above. These actions in turn are thought to collectively result in a decrease in cholesterol synthesis and a reduction in serum cholesterol levels.

General Procedure for the Preparation of Unsaturated 14,15-Substituted Lanosterols

The compounds of the present invention accomodate the necessary requirements for lanosta-8,24-dien-3β-ol 14α-methyl demethylase inhibition and suppression of HMGR activity. To prepare these compounds a three-prong synthetic approach was employed, i.e., monosubstitution at the 14- or 15-position and disubstitution at the 14- and 15-positions.

14-Monosubstitution (SCHEMES I-V)

Introduction of the appropriate substituent at the 14α-position required the elaboration of lanosta-8,24-dien-3β-ol (SCHEME I, Compound 1) into the protected 3β-hydroxy-14α-hydroxymethyl-dihydrolanosterol (Compound 6).

SCHEME I

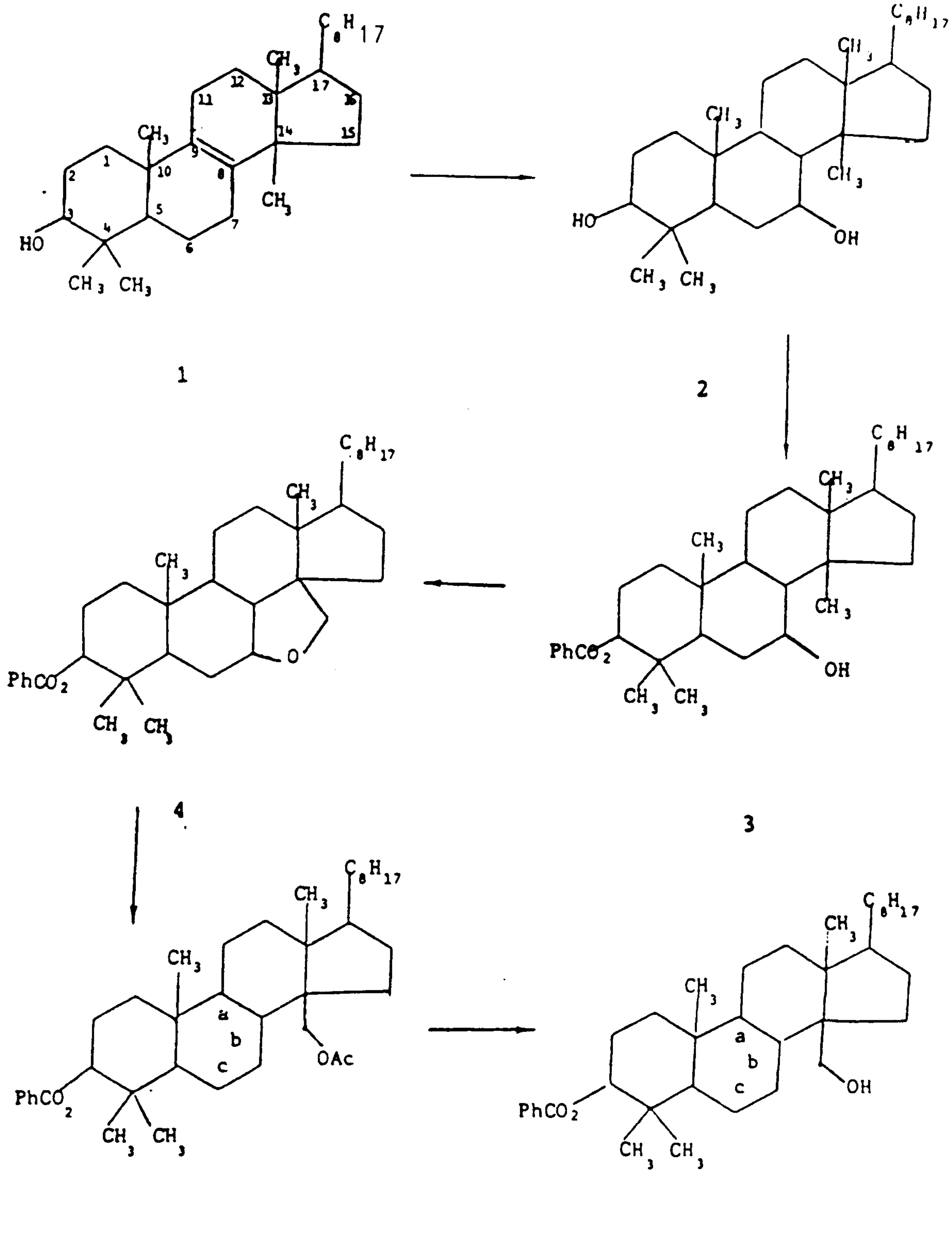

1

2

3

4

5

6

Transformation of the commercially available lanosta-8,24-dien-3$\beta$-ol (obtained from Sigma Chemical Co., P.O. Box 14508, St. Louis, MO 63178) to the diol (Compound 2) was achieved using the procedure described in Parish et al., J. Lipid Res., 22: 859-868 (1981). To utilize this intermediate in our studies, a novel synthetic route that allows selective protection of the 3$\beta$-hydroxy group while the 14$\alpha$-hydroxymethyl group is free for elaboration was employed. Taking advantage of the sterically encumbered secondary alcohol at the 7$\alpha$ position, the diol (Compound 2) was treated with freshly distilled benzoyl chloride in dry

pyridine (obtained from Aldrich Chemical Co., Inc., 940 West St. Paul Ave., Milwaukee, WI 53233) at 40° for 25 min. The reaction resulted, after silica gel chromatography, in the recovery of 3β-benzoate (Compound 3) in a 48% yield. Using the directing effects of the 7α-hydroxyl group, the resulting Compound 3 was oxidized in refluxing benzene under a nitrogen atmosphere by means of recrystallized (from acetic acid) lead tetraacetate (obtained from Aldrich) treatment. After 17 hr of refluxing followed by silica gel chromatography, the desired furan, Compound 4, was obtained in a yield of 70%. Furan ring cleavage of Compound 4 was achieved by exposing 4 to excess pyridine hydrochloride (obtained from Fluka A.G., Buchs, Switzerland) in refluxing acetic anhydride for 18 hr under an atmosphere of nitrogen. Three olefin isomers of the newly generated acetate Compound 5 (Compounds 5a, 5b and 5c) were obtained in an overall 60% yield.

Separation of the isomers by silica gel chromatography (MPLC) provided 5c (16% yield), and separation by high pressure liquid chromotography (HPLC) afforded 5a (19% yield) and 5b (27% yield). With all three olefinic isomers separated, the final selective hydrolysis was achieved by treatment of 5a with ethanolic potassium hydroxide for 2 hr at 10° to generate Compound 6a in 68% yield. In the same manner, both Compounds 6b (73% yield) and 6c (62% yield) were also obtained, providing entry into the double bond isomers of the 14α-substituted series.

Following preparation of the critical monoprotected diol, Compound 6, the next goal is the elaboration of that compound into the desired 14α-substituted dihydrolanosterols. This is shown in SCHEME II.

SCHEME II

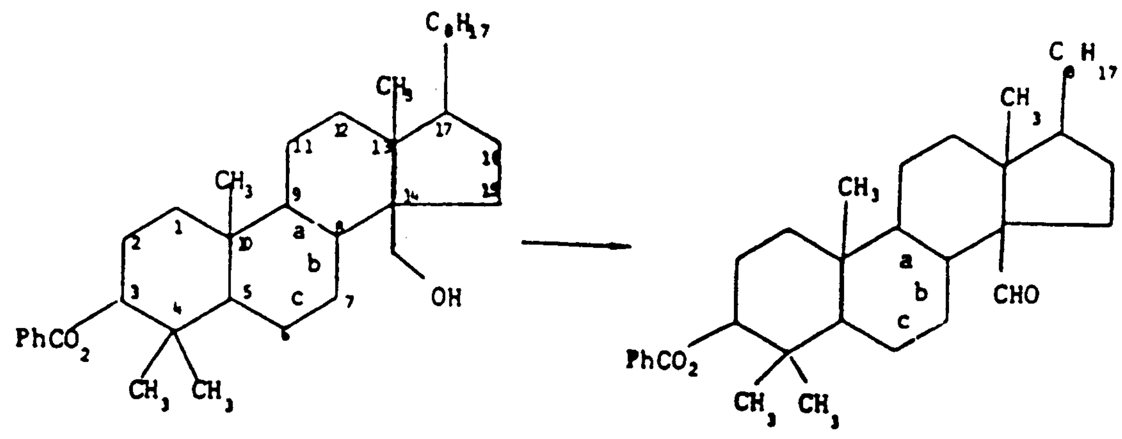

6 7

Oxidation of the 14α-hydroxymethyl group to the 14α-carboxaldehyde Compound 7a was achieved in a 93% yield by treating Compound 6a with Jones reagent (prepared as described in Meinwald et al., Org. Syn., 45:77-79 (1965)) at -10° for 15 min. Compound 7a was then recovered using medium pressure silica gel chromatography (MPLC). In the same manner Compound 7b was prepared in 85% yield. The 14α-carboxyaldehyde Compound 7c was prepared in a 60% yield by treatment at room temperature of a dichloromethane solution of Compound 6c with pyridinium dichromate (Aldrich) and powdered 0.4nm (4A) molecular sieves (Aldrich) followed by silica gel purification on MPLC.

Further elaboration of the 14α position (SCHEME III) was achieved by treating the hydroxy aldehyde Compound 10 (prepared as described by Shafiee et al., J. Lipid Res., 27:1-10 (1986)) with a variety of alkyl and alkenyl anions (Grignards, lithium reagents).

SCHEME III

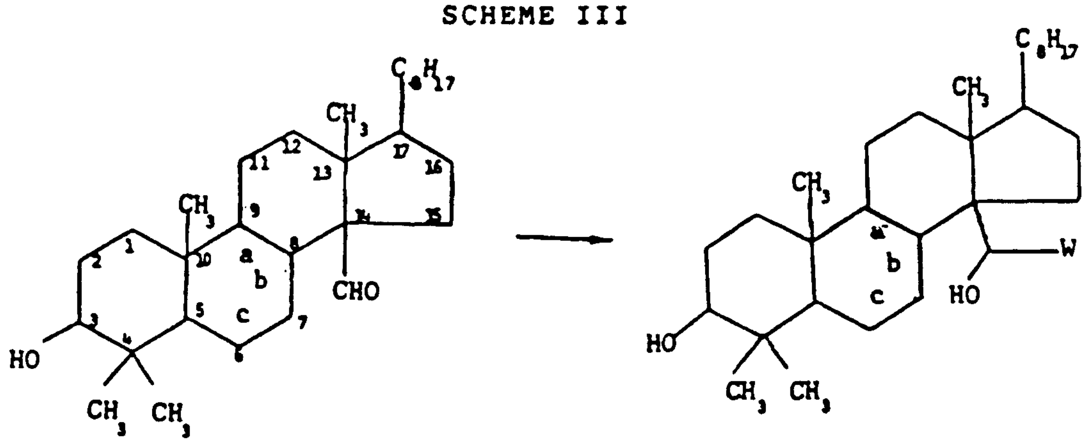

10

11 W = CH=CH$_2$
12 W = CH=CH$_2$

Exposure of an anhydrous tetrahydrofuran solution of Compound 10a to an excess of a vinyl magnesium bromide tetrahydrofuran solution (Aldrich) at room temperature resulted in formation of the diastereomeric diols, Compound 11a and Compound 12a, in 87% combined yield. The diastereomers were separated by MPLC to provide compound 11a in a 66% yield and Compound 12a in a 21% yield. Compounds 11a and 12a are two additional examples of 14α-substituted lanosterols of the present invention. In the same manner, Compounds 11b, 11c, 12b, and 12c may be prepared.

SCHEME V

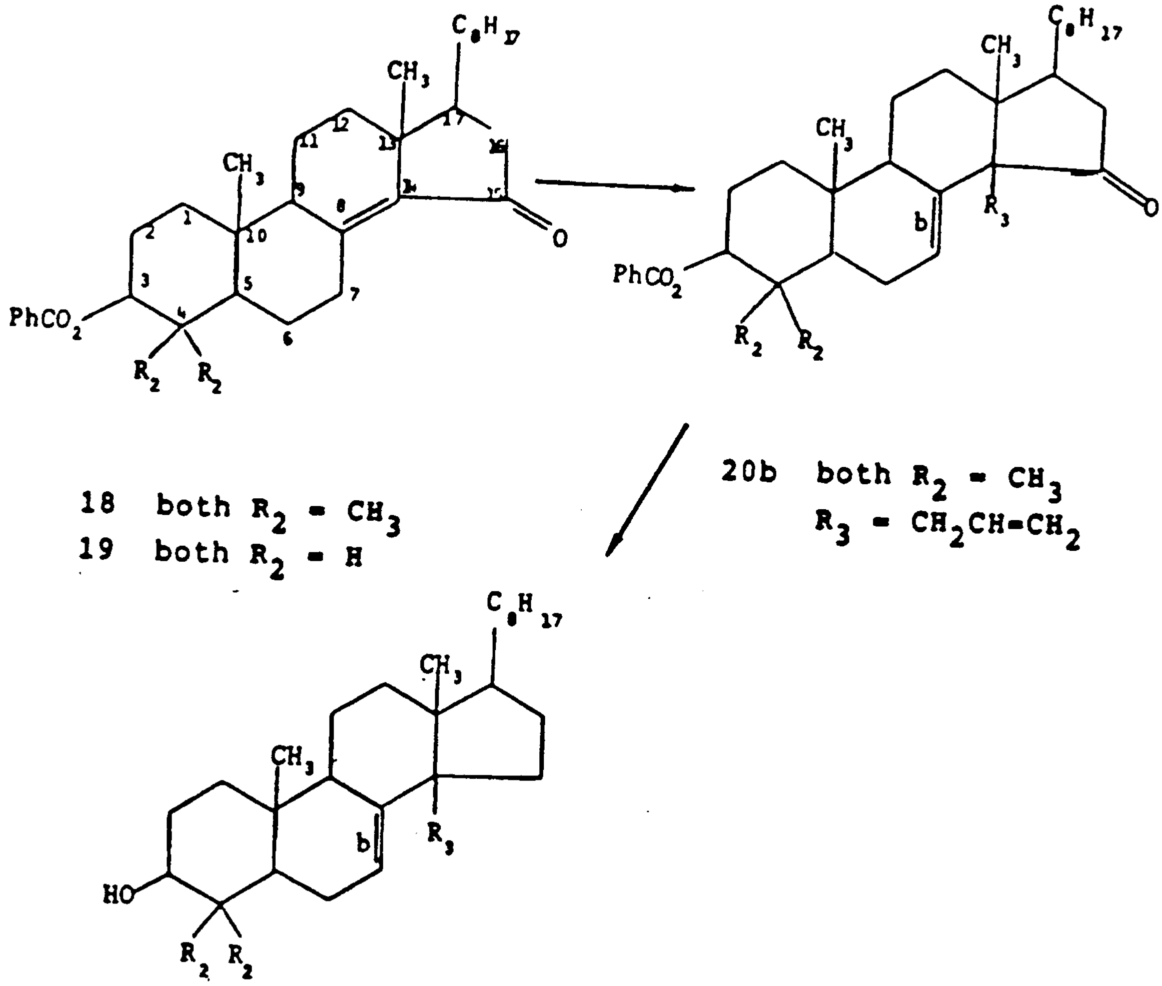

18 both $R_2$ = $CH_3$
19 both $R_2$ = H

20b both $R_2$ = $CH_3$
$R_3$ = $CH_2CH{=}CH_2$

21b both $R_2$ = $CH_3$
$R_3$ = $CH_2CH{=}CH_2$

Compound 18 was alkylated with freshly distilled allyl bromide (Aldrich) in the presence of potassium tertiary butoxide in tertiary butanol to afford, after silica gel chromatography, the 14α-allyl-3β-benzoyloxy-4,4-dimethyl-5α-cholest-7-ene (Compound 20b), in 20% yield (SCHEME V). The 15-ketone was reduced by treating Compound 20b with excess hydrazine in hot (180-220°) diethylene glycol with excess sodium to generate the deprotected 14α-substituted dihydrolanosterol Compound 21b, a Formula I compound, as a mixture of 14α- and 7α-substituted lanosterols. The 14α and 7α compounds can then be separated by chemical chromatographic methods such as argenic chromatography readily known to those skilled in the art. In the same fashion, Compound 21a can also be prepared.

Using the above method, and modifications thereof which would be obvious to those skilled in the art, other examples of the lanosterols series within the scope of the present invention can be prepared.

15-Substitution (Schemes VI-VIIA)

The ketones (SCHEME VIIA), Compounds 29a, 30a and 42a, are prepared by the elaboration of Compounds 38a or 39a.

SCHEME VIIA

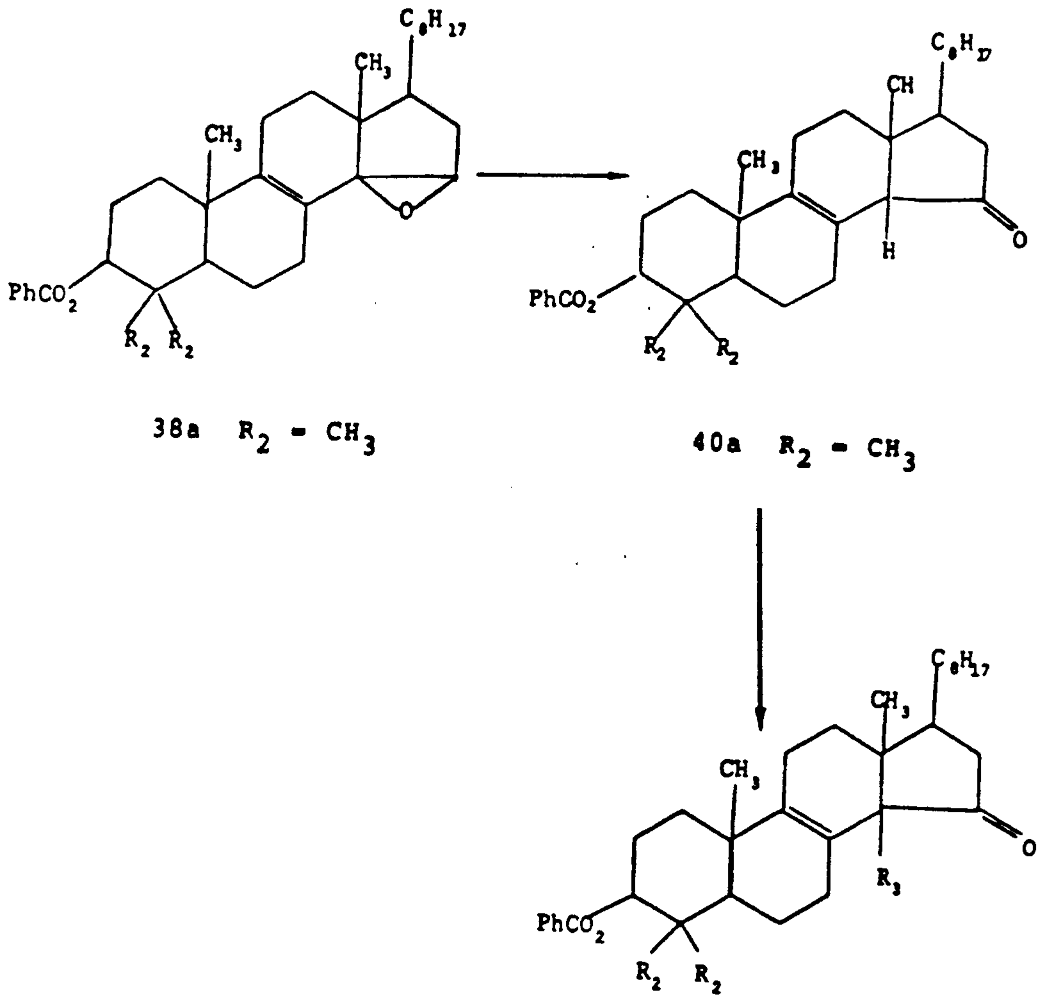

38a $R_2$ = $CH_3$

40a $R_2$ = $CH_3$

29a $R_2$ = $CH_3$ (not according to the invention)

Preparation of epoxide 38a was performed using a process analogous to that described for the preparation of 39a in Anastasia et al., J. Org. Chem. 46: 3265-3267 (1981). Exposure of the epoxide 38a to excess boron trifluoride etherate (Aldrich Chemical Co.) at 0° for 1 hour provided in 75% yield the new ketone 40a with the 14β hydrogen substitution. Alkylation of ketone 40a with methyl iodide in potassium tertiary butoxide in tertiary butanol supplied Compound 29a in 75% yield after recrystallization. Hydrolysis of the benzoate in Compound 29a using 5% potassium hydroxide in ethanol at 80°C for 18 hours provided the hydroxy ketone, Compound 28a. Exposure of Compound 28a to hydroxylamine hydrochloride (Aldrich) in hot pyridine (85°) for 18 hours afforded 15 oxime Compound 31a in 85% yield after recyrstallization. In this manner Compounds 38a, and 39a can be converted into Compounds 30a, and 42a which are included in the scope of the invention. Elaboration of Compounds 29a, 30a, and 42a in the similar fashion as Compounds 24b, 21b, provides entry into other compounds of the present invention. In addition, this process allows 14-15 difunctional modification of Compound 42a to generate additional compounds within the present scope.

## Preparation of Salts

Physiologically acceptable salts of the compounds are also within the scope of the present invention and can be prepared in a number of ways apparent to those skilled in the art.

For example, metal salts can be made by contacting compounds of the invention with a solution of an alkali or alkaline earth metal salt having a sufficiently basic anion (e.g., hydroxide, alkoxide or carbonate). Quaternary amine salts can be made by similar techniques.

Salts can also be prepared by exchange of one cation for another. Cationic exchange can be effected by direct contact of an aqueous solution of a salt of a compound of the invention (e.g. alkali or quaternary amine salt) with a solution containing the cation to be exchanged. This method is most effective when the desired salt containing the exchange cation is insoluble in water and can be separated by filtration.

Exchange may also be effected by passing an aqueous solution of a salt of a compound (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of the present invention with a suitable acid, e.g., p-toluenesulfonic acid, acetic acid or the like.

The preparation of compounds within the scope of the invention is further illustrated by the following specific examples.

Synthetic Examples

The following Examples describe synthetic procedures employed in production of specific compounds within the scope of the present invention. Unless otherwise indicated, all parts and percentages in the following Examples and in the General Procedures set forth above are by weight and all temperatures are reported in degrees Celsius (°C). All chromatography solvents percentages were determined by volume. All proton NMR spectra are referenced to tetramethylsilane (TMS) at 0.00 ppm while all fluorine NMR spectra are referenced to freon®-11 (F-11) at 0.00 ppm.

The following abbreviations are employed in the Examples:

| | |
|---|---|
| NMR: | nuclear magnetic resonance spectroscopy |
| IR: | infrared spectroscopy |
| MS: | mass spectrometry |
| HRMS: | high resolution mass spectrometry |
| EI: | electron impact |
| CI: | chemical ionization |
| EA: | elemental analysis |
| $[\alpha]^{25}$: | optical rotation at 25°C at the sodium D line |
| m.p.: | melting point |
| MPLC: | medium pressure liquid chromatography |
| HPLC: | high pressure liquid chromatography |
| Rf: | retention factor on silica gel thin layer chromatography |
| GC: | gas chromatography |

Particular intermediates or products are identified by reference to the numbered compounds in the general synthetic procedures summarized above. Physical data for various compounds produced by procedures substantially corresponding to the description contained in each Example are provided following the individual

Examples.

Compounds 2-7 are not according to the invention.

Example 1 (A-V):
Preparation of 14,15-Substituted Lanosterols

A. Preparation of 3$\beta$-benzoyloxy-lanost-7$\alpha$-ol (Compound 3)

Lanostane-3$\beta$,7$\alpha$-diol (Compound 2) (4.6 g, 10.3 mmol) was dissolved in anhydrous pyridine (100 mL) at 40°. Benzoyl chloride (6.0 mL, 51.7 mmol) was added and the mixture was stirred at 40° for 25 min. The cooled reaction mixture (0°) was diluted with ice cold other (200 mL) and acidified with 1N HCl to pH 6.5. The organic fraction was washed with 10% aqueous cupric sulfate (2 x 100 mL), water (1 x 50 mL), dried over anhydrous magnesium sulfate and evaporated under reduced pressure to afford the crude residue. The residue was subjected to medium pressure liquid chromatography (0.689 MPa (100 psi), 100 cm X 2.7 cm) using 0.5% ethyl acetate in toluene (4 L) and then 2% ethyl acetate in toluene (fractions: 27 mL). The

contents of fractions 113 through 202 were pooled and evaporated under reduced pressure, providing 2.72 g (48%, corrected 64%) of pure Compound 3.

Physical Data (Compound 3):

$[\alpha]^{25}$ = +25.8° +/- 3.1° (c = 0.64, $CHCl_3$);
m.p. = 190-190.5° (white flakes, acetone);
Rf = 0.55 (10% ethyl acetate in toluene);
NMR (300 MHz $CDCl_3$): 8.06 (d, J = 7.5 Hz, 2H, phenyl), 7.60-7.40 (m, 3H, phenyl), 4.79 (dd, J = 11.4 Hz, 4.5 Hz, 1H, 3-CHOR), 4.11 (s, 1H, 7-CHOH), 2.0-0.85 (m, 27H), 1.11 (s, 3H, $CH_3$), 1.06 (s, 3H, $CH_3$), 1.01 (s, 3H, $CH_3$), 0.93 (s, 3H, $CH_3$), 0.88 (d, J = 6.6Hz, 6H, 26-$CH_3$ and 27-$CH_3$), 0.88 (d, J = 6.5 Hz, 3H, 21-$CH_3$), 0.76 (s, 3H, 18-$CH_3$);
IR ($CHCl_3$ solution, $cm^{-1}$): 3520 (bw, OH), 2950 (s, CH sat), 2870 (s, CH sat), 1710 (s, C=O), 1600(w), 1580(w), 1465(m), 1450(m), 1275(vs), 1115(s);
MS (EI): 550 (3%, M+), 517 (21%, M $-H_2O$, $-CH_3$), 395 (96%, M $-H_2O$, $-CH_3$, $-C_6H_5COOH$);
HRMS for $C_{37}H_{58}O_3$ (M+): calculated 550.4386, found 550.4354.

B. Preparation of 3β-benzoyloxy-7α,32-epoxylanostane (Compound 4)

3β-benzoyloxy-lanost-7α-ol (Compound 3) (2.72 g, 4.95 mmol) was dissolved in benzene (1.1 L). About 150 mL of the solvent was distilled off to remove any traces of water. Lead tetraacetate (12.8 g, 28.9 mmol) (recrystallized from acetic acid) was added and the resulting mixture was refluxed under a nitrogen atmosphere for 17 hours. After cooling to room temperature, the reaction mixture was treated with a 20% aqueous potassium iodide solution (200 mL) then a saturated solution of sodium thiosulfate was added (until the yellow precipitate had dissolved), and the resultant mixture was extracted with diethyl ether (4 x 150 mL). The combined organic extracts were dried over anhydrous magnesium sulfate and evaporated under reduced pressure. The resulting residue was subjected to MPLC (0.241 MPa (35 psi; 50 cm x 2.5 cm) using 5% diethyl ether in toluene as the eluting solvent (fraction size: 28 mL). The content of fractions 17 through 29 were pooled and the solvent was evaporated under reduced pressure, giving 1.90 g of Compound 4 (70% yield).

Physical Data (Compound 4):

$[\alpha]^{25}$ = +41.0° +/- 0.8° (c = 1.01, $CHCl_3$);
m.p. = 225-227° (fine needles, acetone);
Rf = 0.60 (10% ethyl acetate in toluene);
NMR (300 MHz, $CDCl_3$): 8.05 (d, J = 7.2 Hz, 2H, phenyl), 7.64-7.39 (m, 3H, phenyl), 4.75 (dd, J = 11.5 Hz, 4.6 Hz, 1H, 3-CHOR), 4.22 (m, 1H, 7-CHOR), 4.02 (d, J = 7.5 Hz, 1H, 32-$CH_2OR$), 3.38 (d, J = 7.5 Hz, 1H, 32-$CH_2OR$), 2.1-0.8 (m, 26H), 1.03 (s, 3H, 31-$CH_3$), 0.92-0.87 (m, 18H, $CH_3$s);
IR ($CHCl_3$ solution, $cm^{-1}$): 2955 (s, CH sat), 2870 (s, CH sat) 1710 (s, C=O), 1278(vs), 1116(s), 1025(m), 962(m).
HRMS for $C_{36}N_{53}O_2$ (M $-CH_2OH$): calculated 517.4045, found 517.3994;
MS (EI): 517 (38%, M $-CH_2OH$), 403 (64%, M $-CH_2OH-C_8H_{18}$), 395 (100%, M $-CH_2OH-C_6H_5COOH$).

C. Preparation of 32-acetoxy-3β-benzoyloxy-lanost-8-ene (Compound 5a), 32-acetoxy-3β-benzoyloxy-lanost-7-ene (Compound 5b) and 32-acetoxy-3β-benzoyloxy-lanost-6-ene (Compound 5c)

3β-benzoyloxy-7α-32-epoxy-lanostane (Compound 4) (1.90 9, 3.47 mmol) was refluxed for 18 hr in acetic anhydride (380 mL) with pyridine hydrochloride (3.8 g, 32.9 mmol) under nitrogen. After cooling (25°), the mixture was poured into ice-water (400 mL) and stirred for 2 hr. This aqueous mixture was then extracted with diethyl ether (4 x 150 mL) and the combined ether extracts were washed successively with cold (0°) aqueous 5% hydrochloric acid (300 mL), saturated aqueous sodium bicarbonate (8 x 200 mL), water (2 x 100 mL) and brine (100 mL). The extract was dried over anhydrous magnesium sulfate and evaporation was carried out under reduced pressure. The crude product was subjected to MPLC (0.689 MPa (100 psi), 100 cm x 2.5 cm) using toluene (4 L) and then 0.5% ethyl acetate in toluene as the eluting solvent (fraction size: 27 mL). The contents of the following fractions were pooled and evaporated to dryness: (1) fractions 274-311 (Compound 5c), 329 mg (16%); (2) fractions 312-327 (Compound 5a), 249 mg; (3) fractions 366-402 (Compound 5b), 245 mg and (4) fractions 328-365 (Compounds 5a and 5b in a

32:68 mixture), 461 mg. The mixture of Compounds 5a and 5b was resolved by repeating HPLC (2.76 MPa (400 psi), 50 cm x 2.5 cm) using 0.25% ethyl acetate in toluene as the eluting solvent. Total yields for Compound 5a = 19%, Compound 5b = 27% and Compound 5c = 16%.

Physical Data (Compound 5a):

$[\alpha]^{25}$ = +61° +/- 2° (c= 1.00, $CHCl_3$);
m.p. = 109.5-110° (ethanol - 5% water, needles);
Rf = 0.64 (10% ethyl acetate in toluene)
NMR (300 MHz, $CDCl_3$): 8.06 (d, J = 7.2 Hz, 2H, phenyl), 7.60 - 7.40 (m, 3H, phenyl), 4.75 (dd, J = 11.4 Hz, 4.2 Hz, 1H, 3-CHOR), 4.08 (d, J = 10.5 Hz, 1H, 32-$CH_2OR$), 3.97 (d, J = 10.5 Hz, 1H, 32-$CH_2OR$), 2.2-0.85 (m, 26H), 2.06 (s, 3H, acetate), 1.08 (s, 3H, $CH_3$), 1.06 (s, 3H, $CH_3$), 0.97 (s, 3H, 30-$CH_3$), 0.90 (d, J = 6.3 Hz, 3H, 21-$CH_3$), 0.88 (d, J = 6.6 Hz, 6H, 26-$CH_3$ and 27-$CH_3$), 0.73 (s, 3H, 18-$CH_3$);
IR ($CHCl_3$ solution, $cm^{-1}$): 2950 (s, CH sat), 2860 (s, CH sat), 1710 (s, C=O), 1600 (m), 1465(s), 1450(s), 1275 (vs), 1115(s), 1025(s), 980(s), 970(s);
MS (EI): 530 (2%, M -$CH_3CO_2H$), 517 (22%, M -$CH_2OCOCH_3$), 395 (100%; M -$CH_2OCOCH_3$, -$C_6H_5CO_2H$);
HRMS for $C_{37}2_{54}O_2$ (M -$CH_3CO_2H$): calculated 530.4142, found 530.4116.

Physical Data (Compound 5b):

$[\alpha]^{25}$ = +50° +/- 2° (c = 1.03, $CHCl_3$);
m.p. = 154-155° (ethanol - 5% water, needles);
Rf = 0.63 (10% ethyl acetate in toluene);
NMR (300 MHz, $CDCl_3$): 8.06 (d, J = 7.2 Hz, 2H, phenyl), 7.60 - 7.40 (m, 3H, phenyl), 5.25 (d, J = 4.8 Hz, 1H, 7-CH), 4.79 (dd, J = 10.8 Hz, 4.2 Hz, 1H, 3-CHOR), 4.59 (d, J = 10.8 Hz, 1H, 32-$CH_2OR$), 3.73 (d, J = 10.8 Hz, 1H, 32-$CH_2OR$), 2.15 - 0.85 (m, 25H), 1.99 (s, 3H, acetate), 1.13 (s, 3H, 31-$CH_3$), 0.95 (s, 3H, $CH_3$), 0.94 (s, 3H, $CH_3$), 0.90 (d, J = 6.0 Hz, 3H, 21-$CH_3$), 0.88 (d, J = 7.2 Hz, 6H, 26-$CH_3$ and 27-$CH_3$), 0.73 (s, 3H, 18-$CH_3$);
IR ($CHCl_3$ solution, $cm^{-1}$): 2950 (s, CH sat), 2860 (s, CH sat), 1710 (s, C=O), 1600 (m), 1465 (s), 1450(s), 1380(s), 1365(s), 1275(vs), 1115(s), 1025(s), 965(s). MS (EI): 517 (25%, M -$CH_2CO_2CH_3$), 395 (100%, M -$CH_2CO_2CH_3$);
HRMS for $C_{36}H_{50}O_2$ (M -$CH_2CO_2CH_3$): calculated 517.4045, found 517.3999.

Physical Data (Compound 5c):

$[\alpha]^{25}$ = -36.6° +/- 2° (c = 1.01, $CHCl_3$);
m.p. = 141-141.5° (ethanol - 10% water, very fine needles);
Rf = 0.66 (10% ethyl acetate in toluene);
NMR (300 MHz, $CDCl_3$): 8.06 (d, J = 7.2 Hz, 2H, phenyl), 7.60 - 7.40 (m, 3H, phenyl), 5.61 (d, J = 10.2 Hz, 1H, olefinic H), 5.52 (d, J = 10.2 Hz, 1H, olefinic H), 4.77 (dd, J = 11.4 Hz, 4.8 Hz, 1H, 3-CHOR), 4.56 (d, J = 11.5 Hz, 1H, 32-$CH_2OR$), 3.96 (d, J = 11.5 Hz, 1H, 32-$CH_2OR$), 2.42 - 0.85 (m, 24H), 1.99 (s, 3H, acetate), 1.03 (s, 3H, 31-$CH_3$), 0.97 (s, 3H, $CH_3$), 0.91-0.87 (m, 15H, $CH_3$s);
IR ($CHCl_3$ solution, $cm^{-1}$): 2950 (s, CH sat), 2860 (s, CH sat), 1720 (s, C=O), 1600(m), 1470(s), 1450(s), 1385(s), 1370(s), 1315(s), 1275(vs), 1115(s), 1025(s), 970(s). MS (EI): 517 (9%, M -$CH_2OCOCH_3$), 453 (32%, M -$CH_3CO_2H$, -$C_6H_5$), 408 (30%, M -$CH_3CO_2H$, -$C_66_5CO_2H$), 395 (100%, M -$CH_2OCOCH_3$, -$C_6H_5CO_2H$);
HRMS for $C_{36}H_{53}O_2$ (M -$CH_2OCOCH_3$): calculated 517.4045, found 517.4042.

D. Preparation of 3β-benzoyloxy-lanost-8-en-32-ol (Compound 6a)

32-acetoxy-3β-benzoyloxy-lanost-8-ene (Compound 5a) (330 mg, 559 μmol) was dissolved in ethanol (100 mL) and treated with potassium hydroxide (87%, 8.3 g) in ethanol (23 mL) and water (7 mL) at 5°. The mixture was stirred at 10° for 2 hr and quenched with ice water (40 mL). The mixture was then extracted with dichloromethane (3 x 100 mL) with the combined organic fractions dried over anhydrous magnesium sulfate and evaporated under reduced pressure. The residue was subjected to MPLC (0.38 MPa (55 psi), 50 cm x 1.8 cm) using 2% ethyl acetate in toluene as the eluting solvent (fractions 18 mL). The contents of fractions 13 through 35 were pooled, and after evaporation under reduced pressure provided 209 mg (68%) of (Compound 6a).

Physical Data (Compound 6a):

$[\alpha]^{25}$ = +79° +/- 2° (c = 1.02, $CHCl_3$);
m.p. = 167-168.5° (ethanol -5% water);
Rf = 0.50 (10% ethyl acetate in toluene);
NMR (300 MHz, $CDCl_3$): 8.06 (d, J = 7.2 Hz, 2H, phenyl), 7.60-7.40 (m, 3H, phenyl), 4.75 (dd, J = 11.7 Hz, 4.2 Hz, 1H, 3-CHOR), 3.66(dd, J = 10.8 Hz, 9.3 Hz, 1H, 32-$CH_2$OR), 3.25 (dd, J = 10.8 Hz, 3.8 Hz, 1H, C-32-H), 2.15-0.85 (m, 26H), 1.13 (s, 3H, $CH_3$), 1.08 (s, 3H, $CH_3$), 0.98 (s, 3H, 30-$CH_3$), 0.90 (d, J= 6.5 Hz, 3H, 21-$CH_3$), 0.88 (d, J = 6.6 Hz, 6H, 26-$CH_3$ and 27-$CH_3$), 0.72 (s, 3H, 18-$CH_3$).
IR ($CHCl_3$ solution, $cm^{-1}$): 3480 (bw, OH), 2950 (s, CH sat), 2860 (s, CH sat), 1710 (s, C=O), 1460(s), 1445-(s), 1305(s), 1275(vs), 1115(s).
MS (EI): 530 (8%, M -$H_2O$), 518 (42%, M -$CH_2O$), 395 (68%, M -$CH_2OH$, -$C_6H_5CO_2H$), 105 (100%, $C_6H_5CO+$).
HRMS for $C_{37}H_{54}O_2$ (M -$H_2O$): calculated 530.4124, found 530.4162.

Physical Data (Compound 6b; 73% yield from Compound 5b):

$[\alpha]^{25}$ = +51.5° +/- 2.0° (c = 1.01, $CHCl_3$);
m.p. = 209.5-211° (acetone, needles);
Rf = 0.50 (10% ethyl acetate in toluene);
NMR (300 MHz, $CDCl_3$): 8.06 (d, J = 7.5 Hz, 2H, phenyl), 7.60-7.40 (m, 3H, phenyl), 5.39 (m, 1H, 7-CH), 4.75 (dd, J = 4.7 Hz, 11.3 Hz, 1H, 3-CHOR), 3.66 (d, J = 10.2 Hz, 1H, 32-$CH_2$OH), 3.26 (t, J = 10.2 Hz, 1H, 32-$CH_2$OH),2.15-0.85 (m, 26H), 0.96 (s,3H, $CH_3$), 0.89 (d, J = 6.5 Hz, 3H, 21-$CH_3$), 0.87 (d, J = 6.8 Hz, 6H, 26-$CH_3$ and 27-$CH_3$), 0.74 (s, 3H, 18-$CH_3$);
IR ($CHCl_3$ solution, $cm^{-1}$): 3500 (bw, OH), 2940 (s, CH sat), 2860 (s, CH sat), 1705 (s, C=O), 1600(m), 1465(s), 1315(s), 1275(vs), 1115(s), 1020(s), 970(s);
MS (EI): 518 (22%, M -$CH_2O$), 395 (37%, M -$CH_2OH$, -$C_6H_5CO_2H$), 381 (19%, M -$CH_2O$, -$C_6H_5CO_2H$, -$CH_3$), 105 (100%, $C_6H_5CO+$);
HRMS for $C_{36}H_{54}O_2$ (M -$CH_2O$): calculated 518.4124, found 518.4161.

Physical Data (Compound 6c; 62% yield from Compound 5c):

$[\alpha]^{25}$ = -0.9° +/- 2.0° (c = 1.02, $CHCl_3$);
m.p. = 222-223° (ethanol, needles);
Rf = 0.55 (10% ethyl acetate in toluene);
NMR (300 MHz, $CDCl_3$): 8.05 (d, J = 7.5 Hz, 2H, phenyl), 7.60-7.40 (m, 3H, phenyl), 5.85 (d, J = 10.2 Hz, 1H, olefinic H), 5.69 (d, J = 10.2 Hz, 1H, olefinic H), 4.78 (dd, J = 11.6 Hz, 4.7 Hz, 1H, 3-CHOR), 4.20 (d, J = 11.5 Hz, 1H, 32-$CH_2$OH), 3.46 (t, J = 11.5 Hz, 1H, 32-$CH_3$OH), 2.40 (m, 1H), 2.12-0.85 (m, 24H), 1.04 (s, 3H, $CH_3$), 0.98 (s, 3H, $CH_3$), 0.93 (s, 3H, $CH_3$), 0.90-0.84 (m, 12H, $CH_3$s);
IR ($CHCl_3$ solution, $cm^{-1}$): 3690 (w, OH), 3540 (bw, OH) 2960 (s, CH sat), 2870 (s, CH sat), 1710 (s, C=O), 1600(w), 1580 (w), 1470 (m), 1450 (m), 1315 (m), 1280 (vs), 1120 (s);
MS (EI): 530 (5%, M -$H_2O$), 517 (17%, M -$CH_2OH$), 408 (15%, M -$H_2O$, -$C_6H_5COOH$), 403 (8%, M -$CH_2OH$,-$C_8H_{18}$), 395 (100%, M -$CH_2OH$, -$C_5H_6COOH$);
HRMS for $C_{37}H_{54}O_2$ (M -$H_2O$): calculated 530.4124, found 530.4093.

E1. Preparation of 3β-benzoyloxy-lanost-8-en-32-al (Compound 7a)

3β-benzoyloxy-lanost-8-en-32-ol (Compound 6a) (200 mg, 365 μmol) was dissolved in acetone (100 mL) and treated with Jones reagent (2.3 mL) at -10°. The mixture was stirred for 15 min at -10°. The reaction mixture was diluted with water (100 mL) and quickly extracted with toluene (3 x 50 mL). The combined toluene fractions were washed with water (2 x 50 mL), dried over anhydrous magnesium sulfate, and removed by evaporation under reduced pressure. The resulting residue was subjected to MPLC (0.344 MPa (50 psi), 50 cm x 1.8 cm) being eluted with toluene. The procedure resulted in 185 mg (93%) of Compound 7a.

Physical Data (Compound 7a):

$[\alpha]^{25}$ = -243° +/- 4° (c = 1.00, $CHCl_3$);

m.p. = 206-207° (acetone, fine needles);
Rf = 0.60 (toluene);
NMR (300 MHz, $CDCl_3$): 9.47 (s, 1H, 32-CHO), 8.05 (d, J = 7.5 Hz, 2H, phenyl), 7.60-7.40 (m, 3H, phenyl), 4.73 (dd, J = 11.6 Hz, 5.1 Hz, 1H, 3-CHOR), 2.4-0.85 (m, 26H), 1.14 (s, 3H, $CH_3$), 1.07 (s, 3H, $CH_3$), 0.94 (s, 3H, $CH_3$), 0.90 (d, J = 6.3 Hz, 3H, 21-$CH_3$), 0.87 (d, J = 6.6 Hz, 6H, 26-$CH_3$ and 27-$CH_3$), 0.77 (s, 3H, 18-$CH_3$).
IR ($CHCl_3$ solution, $cm^{-1}$): 2940 (s, CH sat), 2860 (s, CH sat), 1710 (s, C=O), 1690 (s, C=O), 1465(s), 1450(s), 1275(vs), 1115(s);
MS (EI): 517 (37%, M -CHO), 395 (100%, M -CHO, -$C_6H_5CO_2H$);
HRMS for $C_{36}6_{53}O_2$ (M -CHO): calculated 517.4046, found 517.4076.

Physical Data (Compound 7b, 85% yield from Compound 6b; reaction time = 1.5 hours at 0°):

$[\alpha]^{25}$ = +46.5° +/- 2° (c = 0.99, $CHCl_3$);
m.p. = 193.5-195.5° (acetone, fine needles);
Rf = 0.6 (toluene);
NMR (300 MHz, $CDCl_3$): 9.66 (s, 1H, 32-CHO), 8.05 (d, J = 7.5 Hz, 2H, phenyl), 7.60-7.40 (m, 3H, phenyl), 5.45 (m, 1H, 7-CH), 4.76 (dd, J = 11.1 Hz, 4.2 Hz, 1H, 3-CHOR), 2.2-0.85 (m, 25H), 1.14 (s, 3H, $CH_3$), 0.98 (s, 3H, $CH_3$), 0.95 (s, 3H, $CH_3$), 0.93 (d, J = 6.5 Hz, 3H, 21-$CH_3$), 0.87 (d, J = 6.6 Hz, 6H, 26-$CH_3$ and 27-$CH_3$), 0.75 (s, 3H, 18-$CH_3$);
IR ($CHCl_3$ solution, $cm^{-1}$): 2950 (s, CH sat), 2860 (s, CH sat), 1705 (s, C=O), 1600(m), 1275(vs), 1115(s), 970(s);
MS (EI): 518 (5%, M -CO), 517 (13%, M -CHO), 395 (68%, M -CHO, -$C_6H_5CO_2H$), 105 (100%, $C_6H_5CO^+$);
HRMS for $C_{36}H_{54}O_2$ (M -CO): calculated 518.4124, found 518.4115.

E2. Preparation of 3β-benzoyloxy-lanost-6-en-32-al (Compound 7c)

3β-benzoyloxy-lanost-6-en-32-ol (Compound 6c) (73 mg, 133 μmole) was dissolved in dichloromethane (distilled from phosphorous pentoxide) (5 mL) and treated with pyridinium dichromate (98%, Aldrich) (74 mg, 193 μmole) and 4 powdered molecular sieves (Aldrich) (72 mg) at room temperature under dry nitrogen atmosphere. After stirring 2 hr, diethyl ether was added (40 mL) and the mixture was filtered through florisil and Celite (obtained from Manville Products Corp., Denver, CO). Evaporation of solvents under reduced pressure gave a residue that was subjected to MPLC (0.483 MPa (70 psi), 50 cm x 1.2 cm) using toluene as the elutant, to afford 42.9 mg (60%) of Compound 7c.

Physical Data (Compound 7c):

$[\alpha]^{25}$ = -26.8° +/- 2.0° (c = 1.01, $CHCl_3$);
m.p. = 175-177° (acetone, very fine needles);
Rf = 0.8 (5% ethyl acetate in toluene);
NMR (300 MHz, $CDCl_3$): 9.97 (s, 1H, 32-CHO), 8.05 (d, J = 7.5 Hz, 2H, phenyl), 7.60-7.40 (m, 3H, phenyl), 5.69 (d, J = 10.7 Hz, 1H, olefinic H), 5.64 (d, J = 10.7 Hz, 1H, olefinic H), 4.74 (dd, J = 11.7 Hz, 4.8 Hz, 1H, 3-CHOR), 2.46 (m, 1H), 2.23-0.85 (m, 23H), 1.03 (s, 3H, $CH_3$), 0.97 (s, 3H, $CH_3$), 0.95-0.90 (m, 12H, $CH_3$s), 0.87 (d, J = 6.6 Hz, 6H, 26-$CH_3$ and 27-$CH_3$);
IR (KBr wafer, $cm^{-1}$): 2950 (s, CH sat), 2870 (s, CH sat), 1718 (s, C=O), 1710 (s, C=O), 1600(w), 1580(w), 1465(m), 1450(m), 1275(vs), 1115(s);
MS (EI) : 546 (1%, M+), 518 (29%, M -CO), 396 (44%, M -CO, -$C_6H_5COOH$), 381 (100%, M -CO, -$C_6H_5COOH$, -CH3);
HRMS for $C_{37}N_{54}O_3$ (M+): calculated 546.4073, found 546.4099.

H. Preparation of both diastereomers of 4,4-dimethyl-14α-(1'-hydroxy-2'-propenyl)-5α-cholest-8-en-3β-ol (Compounds 11a and 12a)

A 1.6 M solution of vinyl magnesium bromide in tetrahydrofuran (1.0 mL, 1.60 mmol) was added to a solution of lanost-8-en-32-al-3β-ol (prepared as described by Shafiee et al., J. Lipid Res., 27:1-10 (1986)) (Compound 10a) (74.5 mg, 168 μmol) in dry (distilled from sodium and benzophenone) tetrahydrofuran (5 mL) at room temperature. After stirring for 120 min at room temperature, saturated ammonium chloride solution (10 mL) was added and the resulting reaction mixture was extracted with diethyl ether (2 x 50 mL).

The combined organic fractions were washed with water (1 x 50 mL) dried over anhydrous magnesium sulfate and the solvent was evaporated under reduced pressure. The residue was subjected to MPLC (0.551 MPa (80 psi), 50 cm x 1.2 cm) being eluted with 5% ethyl acetate in toluene to afford 52.1 mg of Compound 11a (66% yield) and 16.4 mg of Compound 12a (21% yield).

Physical Data (Compound 11a):

$[\alpha]^{25}$ = +54.7° +/- 2.0° (c = 1.05, $CHCl_3$);
m.p. = 155-157° (acetone, prisms);
Rf = 0.6 (50% ethyl acetate in toluene);
NMR (300 MHz, $CDCl_3$): 6.04-5.92 (m, 1H, olefinic CH), 5.22-5.05 (m, 2H, olefinic $CH_2$), 4.11 (m, 1H, 32-CHOH), 3.22 (m, 1H, 3-CHOH), 2.2-0.85 (m, 28H), 1.01 (s, 3H, $CH_3$), 1.00 (s, 3H, $CH_3$), 0.90 (d, J = 6.5 Hz, 3H, 21-$CH_3$), 0.87 (d, J = 6.8 Hz, 6H, 26-$CH_3$ and 27-$CH_3$), 0.82 (s, 3H, 30-$CH_3$ ), 0.71 (s, 3H, 18-$CH_3$);
IR ($CHCl_3$ solution, $cm^{-1}$): 3600 (m, OH), 3470 (bw, OH), 2950 (s, CH sat), 2870 (s, CH sat), 1465 (m), 1375 (m), 1115(m), 990(m), 920(m);
MS (EI): 452 (1%, M -$H_2O$), 437 (1%, M -$H_2O$, -$CH_3$), 413 (100%, M -$C_3H_4OH$), 395 (45%, M -$C_3H_4OH$, -$C_6H_5COOH$);
HRMS for $C_{32}N_{52}O$ (M -$H_2O$): calculated 452.4018, found 452.3999;
EA for $C_{32}H_{54}O_2$: calculated, C 81.64%, E 11.56%; found, C 81.63%, H 11.46%.

Physical Data (Compound 12a):

$[\alpha]^{25}$ = +49.2° +/- 4.8° (c = 0.42, $CHCl_3$);
m.p. = 133-135° (powder);
Rf = 0.5 (50% ethyl acetate in toluene);
NMR (300 MHz, $CDCl_3$): 5.93-5.78 (m, 1H, olefinic CH), 5.16-5.01 (m, 2H, olefinic $CH_2$), 4.10 (m, 1H, 32-CHOH), 3.25 (dd, J = 11.3 Hz, 4.7 Hz, 1H, 3-CHOH), 2.32 (m, 1H), 2.18-0.85 (m, 27H), 1.07 (s, 3H, $CH_3$), 1.02 (s, 3H, $CH_3$), 0.92 (d, J = 6.3 Hz, 3H, 21-$CH_3$), 0.88 (d, J = 6.6 Hz, 6H, 26-$CH_3$ and 27-$CH_3$), 0.84 (s, 3H, 31-$CH_3$), 0.74 (s, 3H, 18-$CH_3$);
IR (KBr wafer, $cm^{-1}$): 3470 (broad, OH), 2950 (s, CH sat), 2930 (s, CH sat), 2865 (s, CH sat), 2855 (s, CH sat), 1470(m), 1375(m), 920(m);
MS (EI): 408 (30%, M -2$H_2O$, -$CH_3$), 395 (100%, M -$C_3H_4OH$, -$H_2O$);
HRMS for $C_{30}N_{47}$ (M -2$H_2O$, -$CH_3$): calculated 408.3756, found 408.3715;
EA for $C_{32}N_{54}O_2$: calculated, C 81.64%, H 11.56%; found, C 81.73%, H 11.60%.

R. Preparation of 14α-allyl-3β-benzoyloxy-4,4-dimethyl-5α-cholest-7-en-15-one (Compound 20b)

3β-benzoyloxy-4,4-dimethyl-5α-cholest-7-en-15-one (Compound 18) (1.13 g, 2.13 mmol) was added to a 0.5 M solution of potassium in tert-butanol (45 mL) under argon at 25°. Freshly distilled allyl bromide (200 µL, 2.30 mmol) was added and the solution stirred for 1 hr. The reaction mixture was quenched with aqueous ammonium chloride (100 mL) and extracted with toluene (3 X 100 mL). The combined organic fractions were washed with water (1 X 100 mL) then saturated aqueous sodium chloride (1 X 100 mL), dried over anhydrous magnesium sulfate, filtered and solvents removed in vacuo. The residues were purified by means of silica gel chromatography (eluant, 3:20:77, ethyl acetate:toluene:hexane) followed by HPLC (eluant, 1.5:20:78.5, ethyl acetate:toluene:hexane). Two fractions resulted: 14α-allyl-3β-benzoyloxy-4,4-dimethyl-5α-cholest-7-en-15-one (Compound 20b) 240 mg (yield 19.7%), Rf = 0.39, and 7α-allyl-3β-benzoyloxy-4,4-dimethyl-5α-cholest-8(14)-en-15-one, 600mg (yield 49.2%), Rf = 0.37 (1:4:15; ethyl acetate:toluene:hexane).

Physical Data (Compound 20b):

NMR (300 MHz, $CDCl_3$): 8.06 (d, 7.4 Hz, 2H, phenyl), 7.55 (m, 1H, phenyl), 7.45 (m, 2H, phenyl), 6.51 (m, 1H, 7-CH), 5.61 (m, 1H, allyl, CH), 4.98 (m, 2H, allyl, $CH_2$), 4.77 (dd, J = 9.4, 4.6 Hz, 1H, 3-CHOR), 2.7-1.1 (m, 23H), 1.15 (s, 3H, $CH_3$), 0.99 (m, 9H, $CH_3$s), 0.91 (s, 3H, $CH_3$), 0.88 (s, 3H, $CH_3$), 0.82 (s, 3H, 18-$CH_3$).
HRMS for $C_{39}N_{56}O_3$ (M+): calculated 572.4229, found 572.4214.

R1. Preparation of 14α-allyl-3β-benzoyloxy-4,4-dimethyl-5α-cholest-8-en-5-one (Compound 20a)

The same preparative procedure as employed for Compound 20b was used, with the following exceptions: Compound 40a was utilized as the starting material, instead of Compound 18; and crude product was crystallized from ethylacetate to give pure 14α-allyl-3β-benzoyloxy-4,4-dimethyl-5α-cholest-8-en-5-one.

Physical Data (Compound 20a):

m.p. = 172-174 ° C (ethyl acetate)
Rf = 0.42 (5% ethyl acetate/hexanes)
NMR (300 MHz, $CDCl_3$): 8.06 (d, J = 7.2 Hz, 2H, phenyl), 7.60-7.40 (m, 3H, phenyl), 7.75 (m, 1H, olefinic CH), 5.01 (m, 2H, olefinic $CH_2$)4.75 (dd, J = 12, 5Hz, 1H, 3-CHOH), 2.61 (dd, J = 20, 9, 1H), 2.5-1.1 (m, 26H), 1.1 (s, 3H, $CH_3$) 1.05 (s, 3H, $CH_3$), 1.00 (m, 6H), 0.9 (d, J = 7Hz, 6H, 26-$CH_3$ and 27-$CH_3$), 0.80 (s, 3H, 18-$CH_3$).

S. Preparation of 14α-allyl-4,4-dimethyl-5α-cholest-7-en-3β-ol (Compound 21b)

Sodium (20 mg, 0.87 mmol) was added to dry (distilled from calcium hydride) diethylene glycol (3 mL) under nitrogen. Compound 20b, 14α-allyl-3β-benzoyloxy-4,4-dimethyl-5α-cholest-7-en-15-one (54 mg, 0.094 mmol) was then added followed by anhydrous hydrazine (1 mL). The solution was heated under a reflux condenser to 180° for 48 hr. The condenser was removed and the excess hydrazine was distilled off at 220°. The solution was allowed to cool to room temperature then diluted with dichloromethane and water. The aqueous phase was neutralized by addition of 10% hydrochloric acid. The mixture was extracted with diohloromethane and the combined organic fractions were dried over anhydrous magnesium sulfate, filtered and the solvents removed in vacuo to give 25 mg of a complex mixture of 14α-allyl-4,4-dimethyl-5α-cholest-7-en-3β-ol (Compound 21b), and 7α-allyl-4,4-dimethyl-5α-cholest-8(14)-3β-ol.

U. Preparation of 3β-hydroxy-lanost-8-en-15-one(Compound 28a) (not according to the invention)

To a solution of 3β-benzoyloxy-lanost-8-en-15-one (Compound 29a) (0.25 g, 0.46 mmol) in toluene (2.5 mL) and methanol (10 mL) was added anhydrous potassium carbonate (0.7 g). After stirring for 4 hours, toluene (2.5 mL) was added followed 2 hours later by the addition of 5% potassium hydroxide in ethanol. The reaction mixture was heated to 80° for 18 hours, then diluted with water (50 mL) and repeatedly extracted with toluene:ether (1:1) (4 X 100 mL). The combined organic extracts were washed with saturated sodium chloride (1 X 100 mL), dired over anhydrous magnesium sulfate, filtered, and concentrated in vacuo. The resultant residue was purified by silica gel chromatography (10% ethyl acetate in hexanes) to afford (180 mg) 3β-hydroxy-lanost-8-en-15-one (Compound 28a) in 89% yield.

U1. Preparation of 14α-allyl-4,4 = dimethyl-5α-cholest-8-en-3β-ol-15-one. (Compound 45a)

The same procedure as used for Compound 28a was employed with the following exceptions: Compound 20a was used as the starting material; and the solution was stirred at 40 ° C for 4 hours instead of 80 ° C for 18 hours. The procedure afforded Compound 45a in a 92% yield.

Physical Data (Compound 45a):

m.p. = 139-140 ° C (ethyl acetate);
Rf = 0.26 (10% ethyl acetate, 20% toluene, 70% hexanes);
NMR (300 MHz, $CDCl_3$): 5.71 (m, 1H, olefinic CH), 4.96 (m, 2H, olefinic $CH_2$), 3.24 (dd, J = 12.5 Hz, 1H, 3-CHOH), 2.57 (dd, J = 20, 9Hz, 1H), 2.4-1.1 (m, 26H), 1.01 (s, 6H, $CH_3$s), 0.97 (d, J = 6Hz, 3H, 21-$CH_3$), 0.89 (d, J = 7Hz, 6H, 26-$CH_3$ and 27-$CH_3$), 0.82 (s, 3H, 4α-$CH_3$), 0.76 (s, 3H, 18-$CH_3$);
MS (El): 468 (10%, m), 427 (100%, m-$C_3H_5$);
HRMS for $C_{32}H_{12}O_2$ ($M^+$): calculated 468.3967, found 468.4087.

V. Preparation of 3β-hydroxy-lanost-8-en-15-oxime (Compound 31a) (not according to the invention)

3β-hydroxy-lanost-8-en-15-one (Compound 28a) (180 mg, 0.41 mmol) was dissolved in anhydrous pyridine (3 mL) then treated with hydroxylamine hydrochloride (300 mg) (Aldrich). The reaction mixture was heated (85°) for 18 hours at which time an additional hydroxylamine hydrochloride (400 mg) sample was

introduced. After quenching the reaction with water (50 mL), and extracting with toluene:ether (1:1) (2 X 100 mL), the conbined organics were washed with 1N hydrochloric acid (2 X 50 mL), water 50 mL), 10% copper sulfate solution (50 mL), water (50 mL), saturated sodium bicarbonate (50 mL), and saturated sodium chloride (50 mL), then dried over anhydrous magnesium sulfate, filtered, and concentrated in vacuo. The residue was crystallized from ethyl acetate in methanol (1:1) to provide 160 mg of pure 3β-hydroxy-lanost-8-en-15-oxime (Compound 31a) in 85% yield.

Physical Data (Compound 31a)

$[a]^{25}$ = + 88.7° +/- 2.0° (c = 1.02, $CHCl_3$)
m.p. = 187-188° (ethyl acetate: methanol, 1:1)
Rf = 0.25 (10% ethyl acetate in hexane)
NMR (300 MHz, $CDCl_3$):6.90 (bs, 1H, NOH), 3.27 (dd, J = 11.1 Hz, 4.5 Hz, 1H, 3-CHOR), 2.77 (m, 2H), 2.30 (m, 2H), 2.10 (m, 2H), 1.80-0.80 (m, 24H), 1.13 (s, 3H, 14-$CH_3$), 1.03 (s, 3H, 19-$CH_3$), 1.02 (s, 3H, 31-$CH_3$), 0.96 (d, J = 6.3 Hz, 3H, 21-$CH_3$), 0.87 (d, J = 6.6 Hz, 6H, 26-$CH_3$ and 27-$CH_3$), 0.84 (s, 3H, 30-$CH_3$), 0.73 (s, 3H, 18-$CH_3$).
IR ($CHCl_3$, $cm^{-1}$ ):3600 (bw, OH), 3300 (vbm, OH), 2960 (vs, CH sat), 1470 (m), 1375 (m), 1035 (m), 940 (m).

V1. Preparation of 14α-allyl-4,4-dimethyl-5α-cholest-8-en-3β-ol-15-oxime (Compound 46a)

The same procedure as used for Compound 31a was employed, with the following exceptions: the reaction mixture was heated at 60°C for 96 hours instead of 85°C for 18 hours; and Compound 45a was utilized as the starting material instead of Compound 28a. compound 46a was provided in 40% yield.

Physical Data (Compound 46a):

m.p.= 116-117°C (ethyl acetate);
Rf= 0.20 (10% ethyl acetate, 20% toluene, 70% hexanes);
NMR (300 MHz, $CDCl_3$): 8.2 (s, 1H, NOH) 5.82 (m, 1H, olefinic CH), 4.96 (m, 2H, olefinic $CH_2$), 3.24 (dd, J=12, 5Hz, 1H, 3-CHOH), 2.82 (dd, J=20, 9Hz, 1H), 2.5-1.1 (m, 26H), 1.02 (s, 6H, $CH_3$s), 0.95 (d, J=6Hz, 3H, 21-$CH_3$) 0.89 (d, J=7Hz, 6H, 26-$CH_3$ and 27-$CH_3$), 0.83 (s, 3H, $CH_3$), 0.72 (s, 3H, 18-$CH_3$);
MS (EI): 465 (30%, M-$H_2O$), 442 (90%, M-$H_2O$ -$C_3H_5$)
HRMS for $C_{32}H_{51}NO$ (M-$H_2O$): calculated 465.3971, found 465.3932.

## Claims

**Claims for the following Contracting States : AT, BE, CH, DE, FR, GB, GR, IT, LI, LU, NL, SE**

1. A compound of the formula:

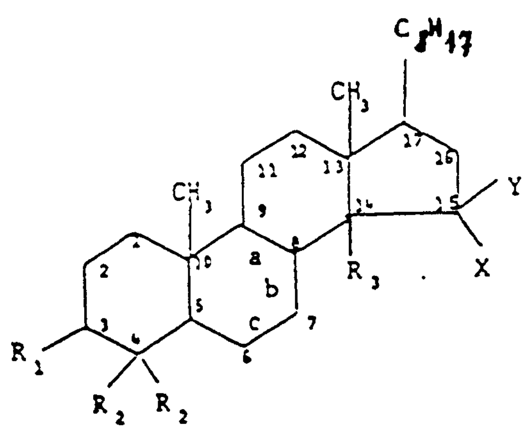

[I]

in which formula the ring structure may be fully saturated or may be unsaturated between one of carbon positions 6-7, 7-8 or 8-9 or between both carbon positions 6-7 and 8-9; and wherein

$R_1$ is =O, $OW_1$, or $OCOW_1$;
$R_2$ is H, $C_1$-$C_6$ alkyl, $C_2$-$C_6$ alkenyl, $C_2$-$C_6$ alkynyl or aryl-$C_1$-$C_6$-alkyl;
$R_3$ is $C_2$-$C_6$ alkenyl, $C_2$-$C_6$ alkynyl, aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$;
$R_4$ is H, $C_1$-$C_6$ alkyl, $C_2$-$C_6$ alkenyl, aryl, aryl-$C_1$-$C_6$-alkyl, or $C_2$-$C_6$ alkynyl;
$R_5$ is $COW_3$, $CSW_3$ or $C(NR_4)W_3$;
X and Y, independently, are H, $C_1$-$C_6$ alkyl, Z, $OR_4$, $OR_5$, $SR_4$, $SR_5$, $N(R_4)_2$, $N(R_5)_2$, $NR_4R_5$, $NR_4OR_4$ or $NR_4OR_5$,
or
X and Y, taken together, are $NR_4$, $NR_5$, $NOR_4$, $NOR_5$, S, $C(R_4)_2$, $C(R_5)_2$, $CR_5R_4$, or O;
Z is halogen;
$W_1$ is H, $C_1$-$C_{20}$ alkyl, $C_2$-$C_{20}$ alkenyl, aryl, aryl-$C_1$-$C_{20}$-alkyl, or $C_2$-$C_{20}$ alkynyl,
$W_3$ is H, $C_1$-$C_6$ alkyl, $C_2$-$C_6$ alkenyl, aryl, aryl-$C_1$-$C_6$-alkyl, $C_2$-$C_6$ alkynyl, $OR_4$ or $N(R_4)_2$;

and their physiologically acceptable salts;
provided that when X is OH, $OR_5$, $N(R_4)_2$, $N(R_5)_2$, $NR_4R_5$, $SR_5$, $NR_4OR_4$, or $NR_4OR_5$, then Y is other than Z, OH, $OR_5$, $SR_5$, $NR_4OR_4$, $NR_4OR_5$, $N(R_4)_2$, or $N(R_5)_2$.

2. A compound of claim 1 wherein
$R_2$ is H, $C_1$-$C_6$ alkyl or aryl-$C_1$-$C_6$-alkyl;
$R_3$ is $C_2$-$C_6$ alkenyl, $C_2$-$C_6$ alkynyl, aryl-$C_1$-$C_6$-alkyl and
X and Y, independently, are H, $C_1$-$C_6$ alkyl, Z, $OR_4$, $OR_5$, $SR_4$, $SR_5$, $N(R_4)_2$, $N(R_5)_2$, $NR_4R_5$, $NR_4OR_4$, or $NR_4OR_5$;
or
X and Y, taken together, are $NR_4$, $NR_5$, $NOR_4$, $NOR_5$, S, $C(R_4)_2$, $C(R_5)_2$, or $CR_5R_4$.

3. A compound of claim 1 wherein $R_2$ is $C_2$-$C_6$ alkenyl, or $C_2$-$C_6$ alkynyl.

4. A compound of claim 1 wherein $R_3$ is $CR_4=CR_4C(R_4)_2Z$, $CR_4=CR_4C(R_4)_2OR_5$, $CR_4=CR_4C(R_4)_2OR_4$.

5. A compound of claim 1 wherein X and Y, taken together, are O.

6. A compound of claim 1 wherein $R_3$ is $C_2$-$C_6$ alkenyl, aryl-$C_1$-$C_6$-alkyl, $C_2$-$C_6$ alkynyl.

7. A compound of claim 1 wherein $R_3$ is $C_2$-$C_6$ alkenyl, aryl-$C_1$-$C_6$-alkyl.

8. A compound of claim 1 wherein
X and Y, independently, are H, $C_1$-$C_6$ alkyl, $OR_4$, $OR_5$, $SR_5$, $SR_4$, $N(R_4)_2$, $N(R_5)_2$, $NR_4R_5$, $NR_4OR_4$, or $NR_4OR_5$;
or
X and Y, taken together, are $NR_4$, $NR_5$, $NOR_4$, $NOR_5$, S, $C(R_4)_2$, $C(R_5)_2$, or $CR_4R_5$.

9. A compound of claim 1 wherein
X and Y, independently, are H, $C_1$-$C_6$ alkyl, Z, $OR_4$, $OR_5$, $SR_5$, $SR_4$, $N(R_4)_2$, $NR_4OR_4$, $NR_4OR_5$, $N(R_5)_2$ or $NR_4R_5$;
or
X and Y, taken together, are $NR_4$, $NR_5$, $NOR_5$, S, $C(R_4)_2$, $C(R_5)_2$, or $CR_4R_5$.

10. A compound of claim 1 wherein
$R_1$ is OH;
$R_2$ is H, or $CH_3$;
$R_3$ is $CH_2CH=CH_2$; and
X and Y, independently, are H, F, or OH;
or
X and Y, taken together, are NOH.

11. A compound of claim 1 wherein

| | |
|---|---|
| $R_1$ | is OH; |
| $R_2$ | is H, or $CH_3$; |
| $R_3$ | is $CH_2CH=CH_2$; and |
| X and Y, | independently, are H or OH. |

**12.** A compound of claim 1 which is 4,4-dimethyl-14α-(1'-hydroxy-2'-propenyl)-5α-cholest-8-en-3β-ol; 14α-allyl-4,4-dimethyl-5α-cholest-8-en-3β-ol-15-oxime.

**13.** A composition suitable for inhibiting lanosterol-8,24-dien-3β-ol 14α-methyl-demethylase activity comprising

**(i)** an effective amount of a compound of the formula

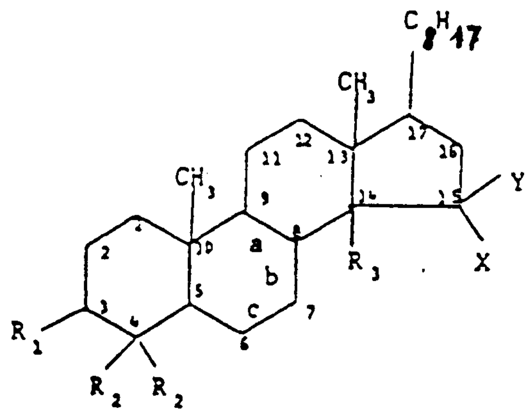

[I]

in which formula the ring structure may be fully saturated or may be unsaturated between one of carbon positions 6-7, 7-8 or 8-9 or between both carbon positions 6-7 and 8-9; and wherein

| | |
|---|---|
| $R_1$ | is =O, $OW_1$, or $OCOW_1$; |
| $R_2$ | is H, $C_1$-$C_6$ alkyl, $C_2$-$C_6$ alkenyl, $C_2$-$C_6$ alkynyl or aryl-$C_1$-$C_6$-alkyl; |
| $R_3$ | is $C_2$-$C_6$ alkenyl, $C_2$-$C_6$ alkynyl, aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$; |
| $R_4$ | is H, $C_1$-$C_6$ alkyl, $C_2$-$C_6$ alkenyl, aryl, aryl-$C_1$-$C_6$-alkyl, or $C_2$-$C_6$ alkynyl; |
| $R_5$ | is $COW_3$, $CSW_3$, or $C(NR_4)W_3$; |
| X and Y, | independently, are H, $C_1$-$C_6$ alkyl, Z, $OR_4$, $OR_5$, $SR_4SR_5$, $N(R_4)_2$, $N(R_5)_2$, $NR_4R_5$, $NR_4OR_4$, $NR_4OR_5$; |
| | or |
| X and Y, | taken together, are $NR_4$, $NR_5$, $NOR_4$, $NOR_5$, S, $C(R_4)_2$, $C(R_5)_2$, $CR_5R_4$, or O; |
| Z | is halogen; |
| $W_1$ | is H, $C_1$-$C_{20}$ alkyl, $C_2$-$C_{20}$ alkenyl, aryl, aryl-$C_1$-$C_{20}$-alkyl, or $C_2$-$C_{20}$ alkynyl; |
| $W_3$ | is H, $C_1$-$C_6$ alkyl, $C_2$-$C_6$ alkenyl, aryl, aryl-$C_1$-$C_6$-alkyl, $C_2$-$C_6$ alkynyl, $OR_4$, or $N(R_4)_2$; |

and their physiologically acceptable salts

provided that when X is OH, $OR_5$, $N(R_4)_2$, $N(R_5)_2$, $NR_4R_5$, $SR_5$, $NR_4OR_4$, or $NR_4OR_5$, then Y is other than Z, OH, $OR_5$, $SR_5$, $NR_4OR_4$, $NR_4OR_5$, $N(R_4)_2$, or $N(R_5)_2$;

and

**(ii)** a pharmaceutically acceptable carrier or diluent.

**14.** A composition suitable for inhibiting 3-hydroxy-3-methylglutaryl coenzyme A reductase activity comprising

**(i)** an effective amount of a compound of the formula

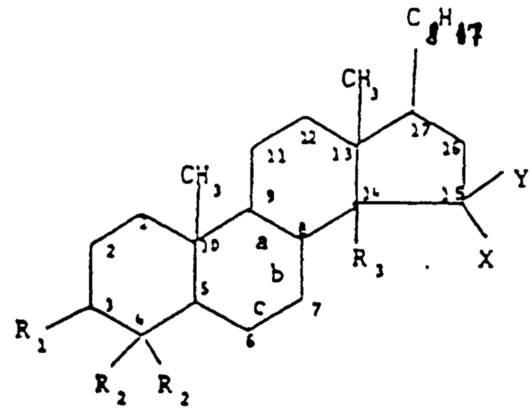

[I]

in which formula the ring structure may be fully saturated or may be unsaturated between one of carbon positions 6-7, 7-8 or 8-9 or between both carbon positions 6-7 and 8-9;
and wherein

| | |
|---|---|
| $R_1$ | is =O, $OW_1$ or $OCOW_1$; |
| $R_2$ | is H, $C_1$-$C_6$ alkyl, $C_2$-$C_6$ alkenyl, $C_2$-$C_6$ alkynyl or aryl-$C_1$-$C_6$-alkyl; |
| $R_3$ | is $C_2$-$C_6$ alkenyl, $C_2$-$C_6$ alkynyl, aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$, |
| $R_4$ | is H, $C_1$-$C_6$ alkyl, $C_2$-$C_6$ alkenyl, aryl, aryl-$C_1$-$C_6$-alkyl, or $C_2$-$C_6$ alkynyl; |
| $R_5$ | is $COW_3$, $CSW_3$ or $C(NR_4)W_3$; |
| X and Y, | independently, are H, $C_1$-$C_6$ alkyl, Z, $OR_4$, $OR_5$, $SR_4$, $SR_5$, $N(R_4)_2$, $N(R_5)_2$, $NR_4R_5$, $NR_4OR_4$ or $NR_4OR_5$, |
| | or |
| X and Y, | taken together, are $NR_4$, $NR_5$, $NOR_4$, $NOR_5$, S, $C(R_4)_2$, $C(R_5)_2$ or $CR_5R_{41}$ or O; |
| Z | is halogen; |
| $W_1$ | is H, $C_1$-$C_{20}$ alkyl, $C_2$-$C_{20}$ alkenyl, aryl, aryl-$C_1$-$C_{20}$-alkyl, or $C_2$-$C_{20}$ alkynyl, |
| $W_3$ | is H, $C_1$-$C_6$ alkyl, $C_2$-$C_6$ alkenyl, aryl, aryl-$C_1$-$C_6$-alkyl, $C_2$-$C_6$ alkynyl, $OR_4$, $N(R_4)_2$; |

and their physiologically acceptable salts;
provided that when X is OH, $OR_5$, $N(R_4)_2$, $N(R_5)_2$, $NR_4R_5$, $SR_5$, $NR_4OR_4$, or $NR_4OR_5$, then Y is other than Z, OH, $OR_5$, $SR_5$, $NR_4OR_4$, $NR_4OR_5$, $N(R_4)_2$, or $N(R_5)_2$;
and
**(ii)** a pharmaceutically acceptable carrier or diluent.

**15.** A composition suitable for decreasing cholesterol formation comprising
**(i)** an effective amount of a compound of the formula

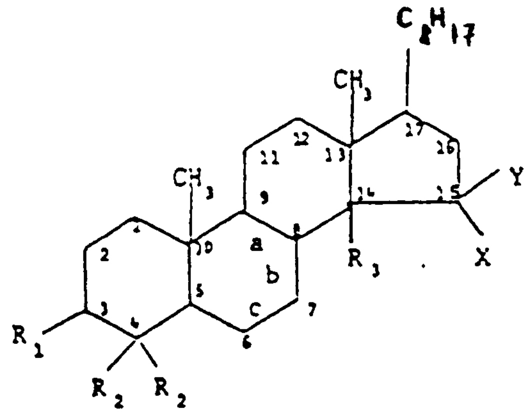

[I]

in which formula the ring structure may be fully saturated or may be unsaturated between one of carbon positions 6-7, 7-8 or 8-9 or between both carbon positions 6-7 and 8-9;
and wherein

| | |
|---|---|
| $R_1$ | is =O, $OW_1$ or $OCOW_1$; |
| $R_2$ | is H, $C_1$-$C_6$ alkyl, $C_2$-$C_6$ alkenyl, $C_2$-$C_6$ alkynyl or aryl-$C_1$-$C_6$-alkyl; |
| $R_3$ | is $C_2$-$C_6$ alkenyl, $C_2$-$C_6$ alkynyl, aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$, |
| $R_4$ | is H, $C_1$-$C_6$ alkyl, $C_2$-$C_6$ alkenyl, aryl, aryl-$C_1$-$C_6$-alkyl, or $C_2$-$C_6$ alkynyl; |
| $R_5$ | is $COW_3$, $CSW_3$ or $C(NR_4)W_3$; |
| X and Y, | independently, are H, $C_1$-$C_6$ alkyl, Z, $OR_4$, $OR_5$, $SR_4$, $SR_5$, $N(R_4)_2$, $N(R_5)_2$, $NR_4R_5$, $NR_4OR_4$ or $NR_4OR_5$, |
| | or |
| X and Y, | taken together, are $NR_4$, $NR_5$, $NOR_4$, $NOR_5$, S, $C(R_4)_2$, $C(R_5)_2$, $CR_5R_4$, or O; |
| Z | is halogen; |
| $W_1$ | is H, $C_1$-$C_{20}$ alkyl, $C_2$-$C_{20}$ alkenyl, aryl, aryl-$C_1$-$C_{20}$-alkyl, or $C_2$-$C_{20}$ alkynyl, |
| $W_3$ | is H, $C_1$-$C_6$ alkyl, $C_2$-$C_6$ alkenyl, aryl, aryl-$C_1$-$C_6$-alkyl, $C_2$-$C_6$ alkynyl, $OR_4$ or $N(R_4)_2$; |

and their physiologically acceptable salts;
provided that when X is OH, $OR_5$, $N(R_4)_2$, $N(R_5)_2$, $NR_4R_5$, $SR_5$, $NR_4OR_4$, or $NR_4OR_5$, then Y is other than Z, OH, $OR_5$, $SR_5$, $NR_4OR_4$, $NR_4OR_5$, $N(R_4)_2$, or $N(R_5)_2$;
and
**(ii)** a pharmaceutically acceptable carrier or diluent.

**16.** A composition suitable for lowering serum cholesterol levels comprising
**(i)** an effective amount of a compound of the formula

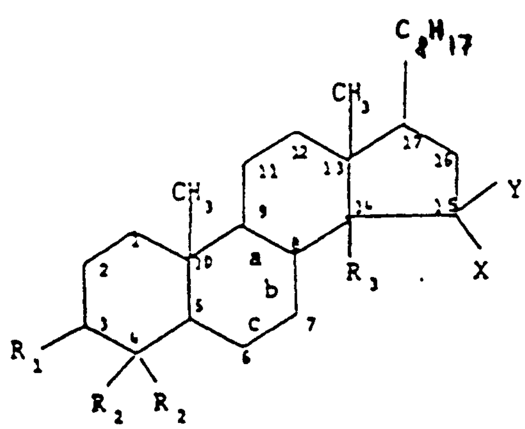

[I]

in which formula the ring structure may be fully saturated or may be unsaturated between one of carbon positions 6-7, 7-8 or 8-9 or between both carbon positions 6-7 and 8-9;
and wherein

| | |
|---|---|
| $R_1$ | is =O, $OW_1$ or $OCOW_1$; |
| $R_2$ | is H, $C_1$-$C_6$ alkyl, $C_2$-$C_6$ alkenyl, $C_2$-$C_6$ alkynyl or aryl-$C_1$-$C_6$-alkyl; |
| $R_3$ | is $C_2$-$C_6$ alkenyl, $C_2$-$C_6$ alkynyl, aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$, |
| $R_4$ | is H, $C_1$-$C_6$ alkyl, $C_2$-$C_6$ alkenyl, aryl, aryl-$C_1$-$C_6$-alkyl, or $C_2$-$C_6$ alkynyl; |
| $R_5$ | is $COW_3$, $CSW_3$ or $C(NR_4)W_3$; |
| X and Y, | independently, are H, $C_1$-$C_6$ alkyl, Z, $OR_4$, $OR_5$, $SR_4$, $SR_5$, $N(R_4)_2$, $N(R_5)_2$, $NR_4R_5$, $NR_4OR_4$ or $NR_4OR_5$, |
| | or |
| X and Y, | taken together, are $NR_4$, $NR_5$, $NOR_4$, $NOR_5$, S, $C(R_4)_2$, $C(R_5)_2$, $CR_5R_4$, or O; |
| Z | is halogen; |
| $W_1$ | is H, $C_1$-$C_{20}$ alkyl, $C_2$-$C_{20}$ alkenyl, aryl, aryl-$C_1$-$C_{20}$-alkyl, or $C_2$-$C_{20}$ alkynyl, |
| $W_3$ | is H, $C_1$-$C_6$ alkyl, $C_2$-$C_6$ alkenyl, aryl, aryl-$C_1$-$C_6$-alkyl, $C_2$-$C_6$ alkynyl, $OR_4$ or $N(R_4)_2$; |

and their physiologically acceptable salts;
provided that when X is OH, $OR_5$, $N(R_4)_2$, $N(R_5)_2$, $NR_4R_5$, $SR_5$, $NR_4OR_4$, or $NR_4OR_5$, then Y is other than Z, OH, $OR_5$, $SR_5$, $NR_4OR_4$, $NR_4OR_5$, $N(R_4)_2$, or $N(R_5)_2$;
and
**(ii)** a pharmaceutically acceptable carrier or diluent.

**Claims for the following Contracting State : ES**

1. A process for producing compounds of the formula:

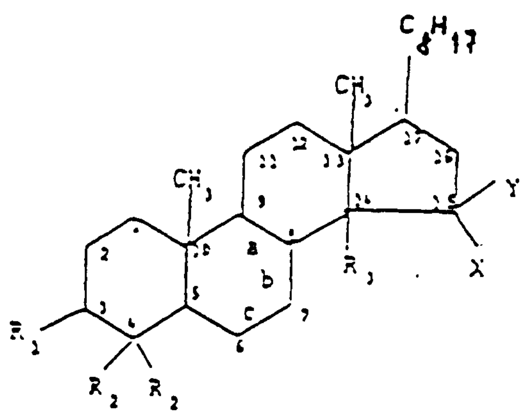

(I)

in which formula the ring structure may be fully saturated or may be unsaturated between one of carbon positions 6-7, 7-8 or 8-9 or between both carbon positions 6-7 and 8-9; and wherein

| | |
|---|---|
| $R_1$ | is =O, $OW_1$, or $OCOW_1$; |
| $R_2$ | is H, $C_1$-$C_6$ alkyl, $C_2$-$C_6$ alkenyl, $C_2$-$C_6$ alkynyl or aryl-$C_1$-$C_6$-alkyl; |
| $R_3$ | is $C_2$-$C_6$ alkenyl, $C_2$-$C_6$ alkynyl, aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$; |
| X and Y, | independently, are H, $C_1$-$C_6$ alkyl, Z, $OR_4$, $OR_5$, $SR_4$, $SR_5$, $N(R_4)_2$, $N(R_5)_2$, $NR_4R_5$, $NR_4OR_4$ or $NR_4OR_5$, |
| | or |
| X and Y, | taken together, are $NR_4$, $NR_5$, $NOR_4$, $NOR_5$, S, $C(R_4)_2$, $C(R_5)_2$, $CR_5R_4$, or O; |
| $R_4$ | is H, $C_1$-$C_6$ alkyl, $C_2$-$C_6$ alkenyl, aryl, aryl-$C_1$-$C_6$-alkyl, or $C_2$-$C_6$ alkynyl; |
| $R_5$ | is $COW_3$, $CSW_3$, or $C(NR_4)W_3$; |
| Z | is halogen; |
| $W_1$ | is H, $C_1$-$C_{20}$ alkyl, $C_2$-$C_{20}$ alkenyl, aryl, aryl-$C_1$-$C_{20}$-alkyl, or $C_2$-$C_{20}$ alkynyl, |
| $W_3$ | is H, $C_1$-$C_6$ alkyl, $C_2$-$C_6$ alkenyl, aryl, aryl-$C_1$-$C_6$-alkyl, $C_2$-$C_6$ alkynyl, $OR_4$ or $N(R_4)_2$; |

and their physiologically acceptable salts;
provided that when X is OH, $OR_5$, $N(R_4)_2$, $N(R_5)_2$, $NR_4R_5$, $SR_5$, $NR_4OR_4$, or $NR_4OR_5$, then Y is other than Z, OH, $OR_5$, $SR_5$, $NR_4OR_4$, $NR_4OR_5$, $N(R_4)_2$, or $N(R_5)_2$;
wherein $R_3$ is aryl-$C_1$-$C_6$-alkyl comprising:
reacting a compound of claim 1 wherein $R_3$ is H or is unsubstituted, with a substituted or unsubstituted alkylating or acylating agent, as appropriate, and, where appropriate, with an oxidizing agent.

**2.** A process for producing a compound of claim 1 wherein
$R_3$ is $C_2$-$C_6$ alkenyl, $C_2$-$C_6$ alkynyl, $CR_4=CR_4C(R_4)_2Z$, $CR_4=CR_4C(R_4)_2OR_5$, $CR_4=CR_4C(R_4)_2OR_4$,
$R_4$ is H, $C_1$-$C_6$ alkyl, $C_2$-$C_6$ alkenyl, aryl, aryl-$C_1$-$C_6$-alkyl, or $C_2$-$C_6$ alkynyl;
comprising: reacting a compound of claim 1 wherein $R_3$ is $R_5$ with a carbanion, and, where appropriate, with a dehydrating agent.

**3.** A process for producing a compound of claim 1 wherein X and Y, taken together, are O
comprising: reacting a compound of the formula

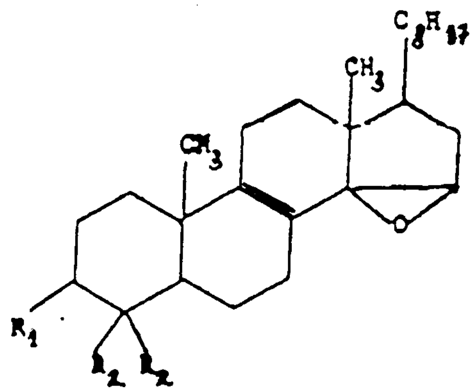

wherein
$R_1$ is =O, $OW_1$, or $OCOW_1$ and
$R_2$ is H, $C_1$-$C_6$ alkyl, $C_2$-$C_6$ alkenyl, $C_2$-$C_6$ alkynyl or aryl-$C_1$-$C_6$-alkyl,
with a lewis acid and, where appropriate, a substituted or unsubstituted alkylating agent.

**4.** A process for producing a compound of claim 1 wherein X and Y, taken together, are $NR_4$, $NR_5$, $NOR_4$, or $NOR_5$,
comprising: reacting a compound of claim 1 wherein X and Y, taken together, are O, with a substituted or unsubstituted amine salt in base.

**5.** A process for producing a compound of claim 1 wherein X and Y, taken together, are S
comprising: reacting a compound of claim 1 wherein X and Y, taken together, are O, with a thiolating agent.

**6.** A process for producing a compound of claim 1 wherein X and Y, independently, are $C_1$-$C_6$ alkyl, or X and Y, taken together, are $C(R_4)_2$, $C(R_5)_2$, $CR_5R_4$,
comprising: reacting a compound of claim 1 wherein X and Y, taken together, are O, with a carbanion, and, where appropriate, a dehydrating agent.

**7.** A process for producing a compound of claim 1 wherein X and Y, independently, are H, $OR_4$, $OR_5$, $SR_4$, $SR_5$, $N(R_4)_2$, $N(R_5)_2$, $NR_4R_5$, $NR_4OR_4$, $NR_4OR_5$,
comprising: reacting a compound of claim 1 wherein X and Y, taken together, are $NR_4$, $NR_5$, $NOR_4$, $NOR_5$, S, or O with a reducing agent.

**8.** A process for producing a compound of claim 1 wherein X and Y, independently, are Z,
comprising: reacting a compound of claim 1 wherein X and Y are, independently, $OR_4$ or $OR_5$, with a halogenating agent.

**Patentansprüche**

**Patentansprüche für folgende Vertragsstaaten : AT, BE, CH, DE, FR, GB, GR, IT, LI, LU, NL, SE**

1. Verbindung der Formel:

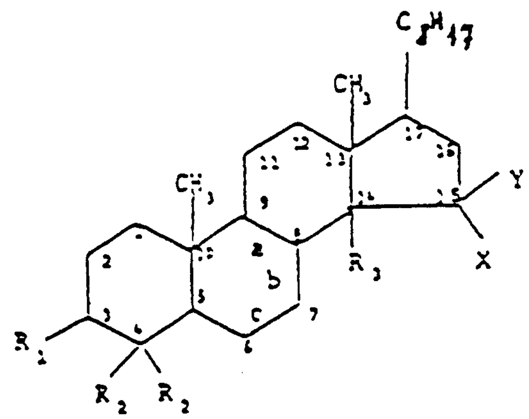

[I]

in der die Ring-Struktur vollständig gesättigt oder zwischen einer der Kohlenstoff-Positionen 6-7, 7-8 oder 8-9 oder zwischen den beiden Kohlenstoff-Positionen 6-7 und 8-9 ungesättigt sein kann; und worin

$R_1$ =O, $OW_1$ oder $OCOW_1$ ist;

$R_2$ H, $C_1$-$C_6$-Alkyl, $C_2$-$C_6$-Alkenyl, $C_2$-$C_6$-Alkinyl oder Aryl-$C_1$-$C_6$-Alkyl ist;

$R_3$ $C_2$-$C_6$-Alkenyl, $C_2$-$C_6$-Alkinyl, Aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$ ist;

$R_4$ H, $C_1$-$C_6$-Alkyl, $C_2$-$C_6$-Alkenyl, Aryl, Aryl-$C_1$-$C_6$-Alkyl, oder $C_2$-$C_6$-Alkinyl ist;

$R_5$ $COW_3$, $CSW_3$ oder $C(NR_4)W_3$ ist;

X und Y, unabhängig voneinander, H, $C_1$-$C_6$-Alkyl, Z, $OR_4$, $OR_5$, $SR_4$, $SR_5$, $N(R_4)_2$, $N(R_5)_2$, $NR_4R_5$, $NR_4OR_4$ oder $NR_4OR_5$ sind,

oder

X und Y, zusammen genommen, $NR_4$, $NR_5$, $NOR_4$, $NOR_5$, S, $C(R_4)_2$, $C(R_5)_2$, $CR_5R_4$ oder O sind;

Z Halogen ist;

$W_1$ H, $C_1$-$C_{20}$-Alkyl, $C_2$-$C_{20}$-Alkenyl, Aryl, Aryl-$C_1$-$C_{20}$-Alkyl oder $C_2$-$C_{20}$-Alkinyl ist,

$W_3$ H, $C_1$-$C_6$-Alkyl, $C_2$-$C_6$-Alkenyl, Aryl, Aryl-$C_1$-$C_6$-Alkyl, $C_2$-$C_6$-Alkinyl, $OR_4$ oder $N(R_4)_2$ ist;

und deren physiologisch annehmbare Salze;

mit der Maßgabe, daß dann, wenn

X OH, $OR_5$, $N(R_4)_2$, $N(R_5)_2$, $NR_4R_5$, $SR_5$, $NR_4OR_4$ oder $NR_4OR_5$ ist,

Y von Z, OH, $OR_5$, $SR_5$, $NR_4OR_4$, $NR_4OR_5$, $N(R_4)_2$ oder $N(R_5)_2$ verschieden ist.

2. Verbindung nach Anspruch 1, worin

$R_2$ H, $C_1$-$C_6$-Alkyl oder Aryl-$C_1$-$C_6$-Alkyl ist;

$R_3$ $C_2$-$C_6$-Alkenyl, $C_2$-$C_6$-Alkinyl, Aryl-$C_1$-$C_6$-Alkyl ist und

X und Y, unabhängig voneinander, H, $C_1$-$C_6$-Alkyl, Z, $OR_4$, $OR_5$, $SR_4$, $SR_5$, $N(R_4)_2$, $N(R_5)_2$, $NR_4R_5$, $NR_4OR_4$ oder $NR_4OR_5$ sind, oder

X und Y, zusammen genommen, $NR_4$, $NR_5$, $NOR_4$, $NOR_5$, S, $C(R_4)_2$, $C(R_5)_2$, oder $CR_5R_4$.

3. Verbindung nach Anspruch 1, worin $R_2$ $C_2$-$C_6$-Alkenyl oder $C_2$-$C_6$-Alkinyl ist.

4. Verbindung nach Anspruch 1, worin $R_3$ $CR_4=CR_4C(R_4)_2Z$, $CR_4=CR_4C(R_4)_2OR_5$, $CR_4=CR_4C(R_4)_2OR_4$ ist.

5. Verbindung nach Anspruch 1, worin X und Y, zusammen genommen, O sind.

6. Verbindung nach Anspruch 1, worin $R_3$ $C_2$-$C_6$-Alkenyl, Aryl-$C_1$-$C_6$-Alkyl, $C_2$-$C_6$-Alkinyl ist.

7. Verbindung nach Anspruch 1, worin $R_3$ $C_2$-$C_6$-Alkenyl, Aryl-$C_1$-$C_6$-Alkyl ist.

8. Verbindung nach Anspruch 1, worin
X und Y, unabhängig voneinander, H, $C_1$-$C_6$-Alkyl, $OR_4$, $OR_5$, $SR_5$, $SR_4$, $N(R_4)_2$, $N(R_5)_2$, $NR_4R_5$, $NR_4OR_4$ oder $NR_4OR_5$ sind; oder
X und Y, zusammen genommen, $NR_4$, $NR_5$, $NOR_4$, $NOR_5$, S, $C(R_4)_2$, $C(R_5)_2$ oder $CR_4R_5$ sind.

9. Verbindung nach Anspruch 1, worin
X und Y, unabhängig voneinander, H, $C_1$-$C_6$-Alkyl, Z, $OR_4$, $OR_5$, $SR_5$, $SR_4$, $N(R_4)_2$, $NR_4OR_4$, $NR_4OR_5$, $N(R_5)_2$, oder $NR_4R_5$ sind; oder
X und Y, zusammen genommen, $NR_4$, $NR_5$, $NOR_5$, S, $C(R_4)_2$, $C(R_5)_2$, oder $CR_4R_5$ sind.

10. Verbindung nach Anspruch 1, worin
$R_1$ OH ist;
$R_2$ H oder $CH_3$ ist;
$R_3$ $CH_2CH=CH_2$ ist; und
X und Y, unabhängig voneinander, H, F oder OH sind; oder
X und Y, zusammen genommen, NOH sind.

11. Verbindung nach Anspruch 1, worin
$R_1$ OH ist;
$R_2$ H oder $CH_3$ ist;
$R_3$ $CH_2CH=CH_2$ ist; und
X und Y, unabhängig voneinander, H oder OH sind.

12. Verbindung nach Anspruch 1, die 4,4-Dimethyl-14α-(1'-hydroxy-2'-propenyl)-5α-cholest-8-en-3β-ol; 14α-Allyl-4,4-dimethyl-5α-cholest-8-en-3β-ol-15-oxim ist.

13. Zur Hemmung der Lanosterol-8,24-dien-3β-ol-14α-methyl-demethylase-Aktivität geeignete Zusammensetzung, umfassend
(i) eine wirksame Menge einer Verbindung der Formel

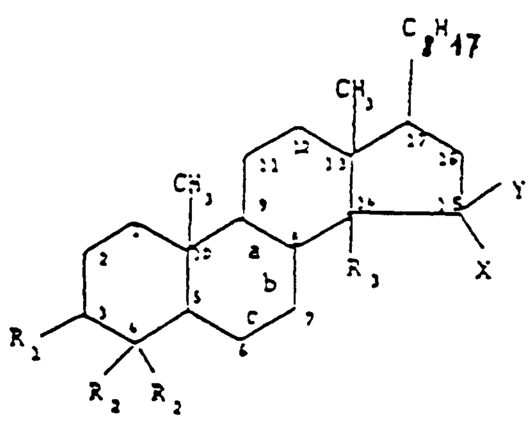

[ I ]

in der die Ring-Struktur vollständig gesättigt oder zwischen einer der Kohlenstoff-Positionen 6-7, 7-8 oder 8-9 oder zwischen den beiden Kohlenstoff-Positionen 6-7 und 8-9 ungesättigt sein kann; und worin
$R_1$ =O, $OW_1$ oder $OCOW_1$ ist;
$R_2$ H, $C_1$-$C_6$-Alkyl, $C_2$-$C_6$-Alkenyl, $C_2$-$C_6$-Alkinyl oder Aryl-$C_1$-$C_6$-Alkyl ist;
$R_3$ $C_2$-$C_6$-Alkenyl, $C_2$-$C_6$-Alkinyl, Aryl-C1-$C_6$-alkyl,$CR_4=CR_4C(R_4)_2Z$, $CR_4=CR_4C(R_4)$-

$_2OR_5$, $CR_4=CR_4C(R_4)_2OR_4$ ist;

$R_4$ H, $C_1$-$C_6$-Alkyl, $C_2$-$C_6$-Alkenyl, Aryl, Aryl-$C_1$-$C_6$-Alkyl, oder $C_2$-$C_6$-Alkinyl ist;

$R_5$ $COW_3$, $CSW_3$ oder $C(NR_4)W_3$ ist;

X und Y, unabhängig voneinander, H, $C_1$-$C_6$-Alkyl, Z, $OR_4$, $OR_5$, $SR_4$, $SR_5$, $N(R_4)_2$, $N(R_5)_2$, $NR_4R_5$, $NR_4OR_4$ oder $NR_4OR_5$ sind, oder

X und Y, zusammen genommen, $NR_4$, $NR_5$, $NOR_4A$, $NOR_5$, S, $C(R_4)_2$, $C(R_5)_2$, $CR_5R_4$ oder O sind;

Z Halogen ist;

$W_1$ H, $C_1$-$C_{20}$-Alkyl, $C_2$-$C_{20}$-Alkenyl, Aryl, Aryl-$C_1$-$C_{20}$-Alkyl oder $C_2$-$C_{20}$-Alkinyl ist,

$W_3$ H, $C_1$-$C_6$-Alkyl, $C_2$-$C_6$-Alkenyl, Aryl, Aryl-$C_1$-$C_6$-Alkyl, $C_2$-$C_6$-Alkinyl, $OR_4$ oder $N(R_4)_2$ ist;

und deren physiologisch annehmbare Salze;

mit der Maßgabe, daß dann, wenn

X OH, $OR_5$, $N(R_4)_2$, $N(R_5)_2$, $NR_4R_5$, $SR_5$, $NR_4OR_4$ oder $NR_4OR_5$ ist,

Y von Z, OH, $OR_5$, $SR_5$, $NR_4OR_4$, $NR_4OR_5$, $N(R_4)_2$ oder $N(R_5)_2$ verschieden ist,

und

(ii) ein pharmazeutisch annehmbares Trägermaterial oder Verdünnungsmittel.

**14.** Zur Hemmung der 3-Hydroxy-3-methylglutaryl-Coenzym A-Reduktase-Aktivitätgeeignete Zusammensetzung, umfassend

(i) eine wirksame Menge einer Verbindung der Formel

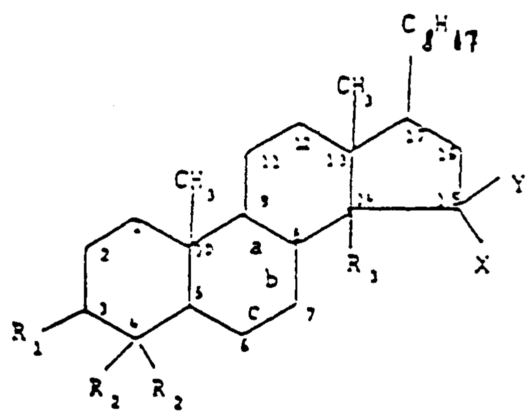

[I]

in der die Ring-Struktur vollständig gesättigt oder zwischen einer der Kohlenstoff-Positionen 6-7, 7-8 oder 8-9 oder zwischen den beiden Kohlenstoff-Positionen 6-7 und 8-9 ungesättigt sein kann; und worin

$R_1$ =O, $OW_1$ oder $OCOW_1$ ist;

$R_2$ H, $C_1$-$C_6$-Alkyl, $C_2$-$C_6$-Alkenyl, $C_2$-$C_6$-Alkinyl oder Aryl-$C_1$-$C_6$-Alkyl ist;

$R_3$ $C_2$-$C_6$-Alkenyl, $C_2$-$C_6$-Alkinyl, Aryl-C1-$C_6$-alkyl,$CR_4=CR_4C(R_4)_2Z$, $CR_4=CR_4C(R_4)_2OR_5$, $CR_4=CR_4C(R_4)_2OR_4$ ist;

$R_4$ H, $C_1$-$C_6$-Alkyl, $C_2$-$C_6$-Alkenyl, Aryl, Aryl-$C_1$-$C_6$-Alkyl, oder $C_2$-$C_6$-Alkinyl ist;

$R_5$ $COW_3$, $CSW_3$ oder $C(NR_4)W_3$ ist;

X und Y, unabhängig voneinander, H, $C_1$-$C_6$-Alkyl, Z, $OR_4$, $OR_5$, $SR_4$, $SR_5$, $N(R_4)_2$, $N(R_5)_2$, $NR_4R_5$, $NR_4OR_4$ oder $NR_4OR_5$ sind, oder

X und Y, zusammen genommen, $NR_4$, $NR_5$, $NOR_4$, $NOR_5$, S, $C(R_4)_2$, $C(R_5)_2$, $CR_5R_4$ oder O sind;

Z Halogen ist;

$W_1$ H, $C_1$-$C_{20}$-Alkyl, $C_2$-$C_{20}$-Alkenyl, Aryl, Aryl-$C_1$-$C_{20}$-Alkyl oder $C_2$-$C_{20}$-Alkinyl ist,

$W_3$ H, $C_1$-$C_6$-Alkyl, $C_2$-$C_6$-Alkenyl, Aryl, Aryl-$C_1$-$C_6$-Alkyl, $C_2$-$C_6$-Alkinyl, $OR_4$ oder $N(R_4)_2$ ist;

und deren physiologisch annehmbare Salze;
mit der Maßgabe, daß dann, wenn
X OH, $OR_5$, $N(R_4)_2$, $N(R_5)_2$, $NR_4R_5$, $SR_5$, $NR_4OR_4$ oder $NR_4OR_5$ ist,
Y von Z, OH, $OR_5$, $SR_5$, $NR_4OR_4$, $NR_4OR_5$, $N(R_4)_2$ oder $N(R_5)_2$ verschieden ist,
und
(ii) ein pharmazeutisch annehmbares Trägermaterial oder Verdünnungsmittel.

**15.** Zur Verminderung der Cholesterin-Bildung geeignete Zusammensetzung, umfassend
(i) eine wirksame Menge einer Verbindung der Formel

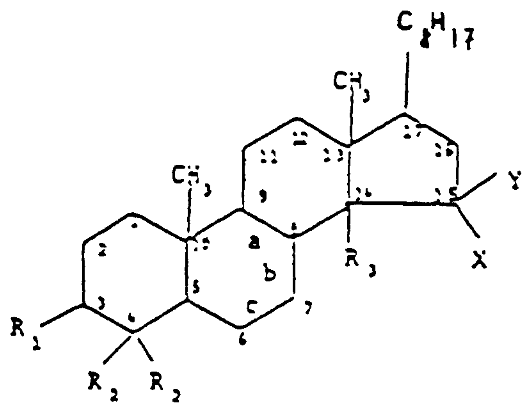

[ I ]

in der die Ring-Struktur vollständig gesättigt oder zwischen einer der Kohlenstoff-Positionen 6-7, 7-8 oder 8-9 oder zwischen den beiden Kohlenstoff-Positionen 6-7 und 8-9 ungesättigt sein kann; und worin

| | |
|---|---|
| $R_1$ | =O, $OW_1$ oder $OCOW_1$ ist; |
| $R_2$ | H, $C_1$-$C_6$-Alkyl, $C_2$-$C_6$-Alkenyl, $C_2$-$C_6$-Alkinyl oder Aryl-$C_1$-$C_6$-Alkyl ist; |
| $R_3$ | $C_2$-$C_6$-Alkenyl, $C_2$-$C_6$-Alkinyl, Aryl-C1-$C_6$-alkyl,$CR_4=CR_4C(R_4)_2Z$, $CR_4=CR_4C(R_4)_2OR_5$, $CR_4=CR_4C(R_4)_2OR_4$ ist; |
| $R_4$ | H, $C_1$-$C_6$-Alkyl, $C_2$-$C_6$-Alkenyl, Aryl, Aryl-$C_1$-$C_6$-Alkyl, oder $C_2$-$C_6$-Alkinyl ist; |
| $R_5$ | $COW_3$, $CSW_3$ oder $C(NR_4)W_3$ ist; |
| X und Y, | unabhängig voneinander, H, $C_1$-$C_6$-Alkyl, Z, $OR_4$, $OR_5$, $SR_4$, $SR_5$, $N(R_4)_2$, $N(R_5)_2$, $NR_4R_5$, $NR_4OR_4$ oder $NR_4OR_5$ sind, oder |
| X und Y, | zusammen genommen, $NR_4$, $NR_5$, $NOR_4$, $NOR_5$, S, $C(R_4)_2$, $C(R_5)_2$, $CR_5R_4$ oder O sind; |
| Z | Halogen ist; |
| $W_1$ | H, $C_1$-$C_{20}$-Alkyl, $C_2$-$C_{20}$-Alkenyl, Aryl, Aryl-$C_1$-$C_{20}$-Alkyl oder $C_2$-$C_{20}$-Alkinyl ist, |
| $W_3$ | H, $C_1$-$C_6$-Alkyl, $C_2$-$C_6$-Alkenyl, Aryl, Aryl-$C_1$-$C_6$-Alkyl, $C_2$-$C_6$-Alkinyl, $OR_4$ oder $N(R_4)_2$ ist; |

und deren physiologisch annehmbare Salze;
mit der Maßgabe, daß dann, wenn
X OH, $OR_5$, $N(R_4)_2$, $N(R_5)_2$, $NR_4R_5$, $SR_5$, $NR_4OR_4$ oder $NR_4OR_5$ ist,
Y von Z, OH, $OR_5$, $SR_5$, $NR_4OR_4$, $NR_4OR_5$, $N(R_4)_2$ oder $N(R_5)_2$ verschieden ist,
und
(ii) ein pharmazeutisch annehmbares Trägermaterial oder Verdünnungsmittel.

**16.** Zur Senkung der Serum-Cholesterin-Werte geeignete Zusammensetzung, umfassend
(i) eine wirksame Menge einer Verbindung der Formel

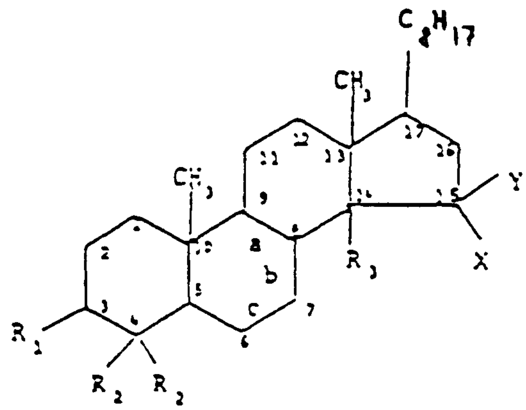

[I]

in der die Ring-Struktur vollständig gesättigt oder zwischen einer der Kohlenstoff-Positionen 6-7, 7-8 oder 8-9 oder zwischen den beiden Kohlenstoff-Positionen 6-7 und 8-9 ungesättigt sein kann; und worin

| | |
|---|---|
| $R_1$ | $=O$, $OW_1$ oder $OCOW_1$ ist; |
| $R_2$ | H, $C_1$-$C_6$-Alkyl, $C_2$-$C_6$-Alkenyl, $C_2$-$C_6$-Alkinyl oder Aryl-$C_1$-$C_6$-Alkyl ist; |
| $R_3$ | $C_2$-$C_6$-Alkenyl, $C_2$-$C_6$-Alkinyl, Aryl-C1-$C_6$-alkyl,$CR_4=CR_4C(R_4)_2Z$, $CR_4=CR_4C(R_4)$-$_2OR_5$, $CR_4=CR_4C(R_4)_2OR_4$ ist; |
| $R_4$ | H, $C_1$-$C_6$-Alkyl, $C_2$-$C_6$-Alkenyl, Aryl, Aryl-$C_1$-$C_6$-Alkyl, oder $C_2$-$C_6$-Alkinyl ist; |
| $R_5$ | $COW_3$, $CSW_3$ oder $C(NR_4)W_3$ ist; |
| X und Y, | unabhängig voneinander, H, $C_1$-$C_6$-Alkyl, Z, $OR_4$, $OR_5$, $SR_4$, $SR_5$, $N(R_4)_2$, $N(R_5)_2$, $NR_4R_5$, $NR_4OR_4$ oder $NR_4OR_5$ sind, |
| | oder |
| X und Y, | zusammen genommen, $NR_4$, $NR_5$, $NOR_4$, $NOR_5$, S, $C(R_4)_2$, $C(R_5)_2$, $CR_5R_4$ oder O sind; |
| Z | Halogen ist; |
| $W_1$ | H, $C_1$-$C_{20}$-Alkyl, $C_2$-$C_{20}$-Alkenyl, Aryl, Aryl-$C_1$-$C_{20}$-Alkyl oder $C_2$-$C_{20}$-Alkinyl ist, |
| $W_3$ | H, $C_1$-$C_6$-Alkyl, $C_2$-$C_6$-Alkenyl, Aryl, Aryl-$C_1$-$C_6$-Alkyl, $C_2$-$C_6$-Alkinyl, $OR_4$ oder N-$(R_4)_2$ ist; |

und deren physiologisch annehmbare Salze;
mit der Maßgabe, daß dann, wenn

| | |
|---|---|
| X | OH, $OR_5$, $N(R_4)_2$, $N(R_5)_2$, $NR_4R_5$, $SR_5$, $NR_4OR_4$ oder $NR_4OR_5$ ist, |
| Y | von Z, OH, $OR_5$, $SR_5$, $NR_4OR_4$, $NR_4OR_5$, $N(R_4)_2$ oder $N(R_5)_2$ verschieden ist, |

und
(ii) ein pharmazeutisch annehmbares Trägermaterial oder Verdünnungsmittel.

**Patentansprüche für folgenden Vertragsstaat : ES**

1. Verfahren zur Herstellung von Verbindungen der Formel:

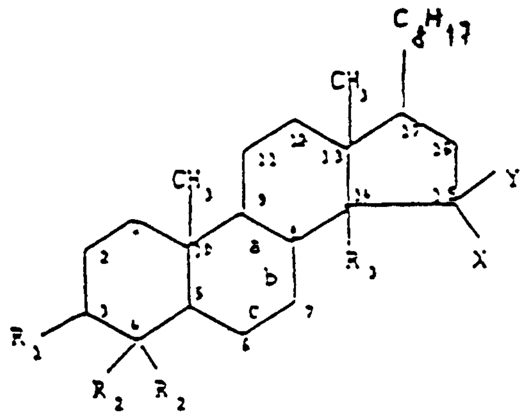

(I)

in der die Ring-Struktur vollständig gesättigt oder zwischen einer der Kohlenstoff-Positionen 6-7, 7-8 oder 8-9 oder zwischen den beiden Kohlenstoff-Positionen 6-7 und 8-9 ungesättigt sein kann; und worin

| | |
|---|---|
| $R_1$ | $=O$, $OW_1$ oder $OCOW_1$ ist; |
| $R_2$ | H, $C_1$-$C_6$-Alkyl, $C_2$-$C_6$-Alkenyl, $C_2$-$C_6$-Alkinyl oder Aryl-$C_1$-$C_6$-Alkyl ist; |
| $R_3$ | $C_2$-$C_6$-Alkenyl, $C_2$-$C_6$-Alkinyl, Aryl-$C_1$-$C_6$-alkyl, $CR_4=CR_4C(R_4)_2Z$, $CR_4=CR_4C(R_4)_2OR_5$, $CR_4=CR_4C(R_4)_2OR_4$ ist; |
| X und Y, | unabhängig voneinander, H, $C_1$-$C_6$-Alkyl, Z, $OR_4$, $OR_5$, $SR_4$, $SR_5$, $N(R_4)_2$, $N(R_5)_2$, $NR_4R_5$, $NR_4OR_4$ oder $NR_4OR_5$ sind, oder |
| X und Y, | zusammen genommen, $NR_4$, $NR_5$, $NOR_4$, $NOR_5$, S, $C(R_4)_2$, $C(R_5)_2$, $CR_5R_4$ oder O sind; |
| $R_4$ | H, $C_1$-$C_6$-Alkyl, $C_2$-$C_6$-Alkenyl, Aryl, Aryl-$C_1$-$C_6$-Alkyl, oder $C_2$-$C_6$-Alkinyl ist; |
| $R_5$ | $COW_3$, $CSW_3$ oder $C(NR_4)W_3$ ist; |
| Z | Halogen ist; |
| $W_1$ | H, $C_1$-$C_{20}$-Alkyl, $C_2$-$C_{20}$-Alkenyl, Aryl, Aryl-$C_1$-$C_{20}$-Alkyl oder $C_2$-$C_{20}$-Alkinyl ist, |
| $W_3$ | H, $C_1$-$C_6$-Alkyl, $C_2$-$C_6$-Alkenyl, Aryl, Aryl-$C_1$-$C_6$-Alkyl, $C_2$-$C_6$-Alkinyl, $OR_4$ oder $N(R_4)_2$ ist; |

und physiologisch annehmbarer Salze derselben;
mit der Maßgabe, daß dann, wenn

X OH, $OR_5$, $N(R_4)_2$, $N(R_5)_2$, $NR_4R_5$, $SR_5$, $NR_4OR_4$ oder $NR_4OR_5$ ist,

Y von Z, OH, $OR_5$, $SR_5$, $NR_4OR_4$, $NR_4OR_5$, $N(R_4)_2$ oder $N(R_5)_2$ verschieden ist,

umfassend die Umsetzung einer Verbindung der Formel I, in der $R_3$ H ist oder die unsubstituiert ist, mit einem geeigneten substituierten oder unsubstituierten Alkylierungs- oder Acylierungsmittel und gegebenenfalls mit einem Oxidationsmittel.

**2.** Verfahren zur Herstellung einer Verbindung nach Anspruch 1, in der

$R_3$ $C_2$-$C_6$-Alkenyl, $C_2$-$C_6$-Alkinyl, $CR_4=CR_4C(R_4)_2Z$, $CR_4=CR_4C(R_4)_2OR_5$, $CR_4=CR_4C(R_4)_2OR_4$ ist;

$R_4$ H, $C_1$-$C_6$-Alkyl, $C_2$-$C_6$-Alkenyl, Aryl, Aryl-$C_1$-$C_6$-Alkyl, oder $C_2$-$C_6$-Alkinyl ist;

umfassend die Umsetzung einer Verbindung des Anspruchs 1, in der $R_3$ $R_5$ ist, mit einem Carbanion und gegebenenfalls mit einem wasserabspaltenden Mittel.

**3.** Verfahren zur Herstellung einer Verbindung nach Anspruch 1, in der
X und Y, zusammen genommen, O sind,
umfassend die Umsetzung einer Verbindung der Formel

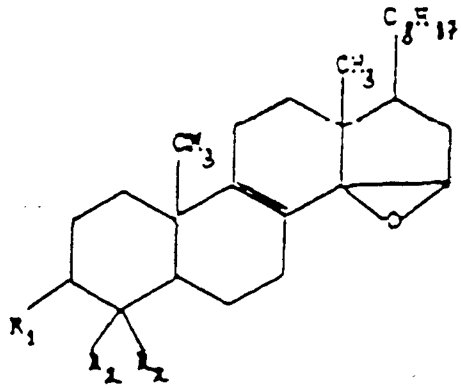

in der

$R_1$ = O,O$W_1$ oder OCO$W_1$ ist;

$R_2$ H, $C_1$-$C_6$-Alkyl, $C_2$-$C_6$-Alkenyl, $C_2$-$C_6$-Alkinyl oder Aryl-$C_1$-$C_6$-Alkyl ist,

mit einer Lewis-Säure und gegebenenfalls mit einem substituierten oder unsubstituierten Alkylierungsmittel.

**4.** Verfahren zur Herstellung einer Verbindung nach Anspruch 1, in der X und Y, zusammen genommen, $NR_4$, $NR_5$, $NOR_4$ oder $NOR_5$ sind, umfassend die Umsetzung einer Verbindung des Anspruchs 1, in der X und Y, zusammen genommen, O sind, mit einem substituierten oder unsubstituierten Amin-Salz in einer Base.

**5.** Verfahren zur Herstellung einer Verbindung nach Anspruch 1, in der X und Y, zusammen genommen, S sind, umfassend die Umsetzung einer Verbindung des Anspruchs 1, in der X und Y, zusammen genommen, O sind, mit einem Thiolierungsmittel.

**6.** Verfahren zur Herstellung einer Verbindung nach Anspruch 1, in der X und Y, unabhängig voneinander $C_1$-$C_6$-Alkyl sind oder X und Y, zusammen genommen, $C(R_4)_2$, $C(R_5)_2$, $CR_5R_4$ sind, umfassend die Umsetzung einer Verbindung des Anspruchs 1, in der X und Y, zusammen genommen, O sind, mit einem Carbanion und gegebenenfalls mit einem wasserabspaltenden Mittel.

**7.** Verfahren zur Herstellung einer Verbindung nach Anspruch 1, in der X und Y, unabhängig voneinander H, $OR_4$, $OR_5$, $SR_4$, $SR_5$, $N(R_4)_2$, $N(R_5)_2$, $NR_4R_5$, $NR_4OR_4$ oder $NR_4OR_5$ sind, umfassend die Umsetzung einer Verbindung des Anspruchs 1, in der X und Y, zusammen genommen, $NR_4$, $NR_5$, $NOR_4$, $NOR_5$, S oder O sind, mit einem Reduktionsmittel.

**8.** Verfahren zur Herstellung einer Verbindung nach Anspruch 1, in der X und Y, unabhängig voneinander Z sind, umfassend die Umsetzung einer Verbindung des Anspruchs 1, in der X und Y, unabhängig voneinander $OR_4$ oder $OR_5$ sind, mit einem Halogenierungsmittel.

**Revendications**

**Revendications pour les Etats contractants suivants : AT, BE, CH, DE, FR, GB, GR, IT, LI, LU, NL, SE**

**1.** Composé de formule:

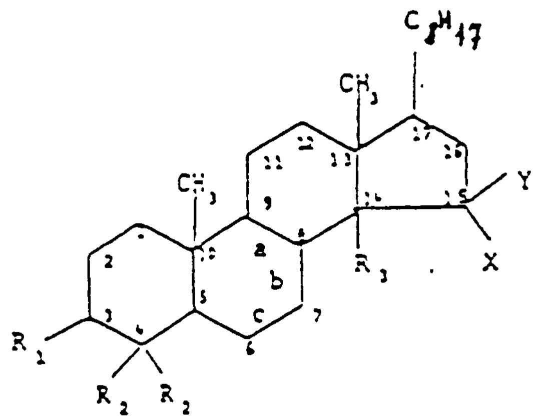

[ I ]

dans laquelle la structure cyclique peut être complètement saturée ou peut être insaturée entre l'une des positions des atomes de carbone 6-7, 7-8 ou 8-9 ou entre les deux positions des atomes de carbone 6-7 et 8-9;
et dans laquelle:

- $R_1$ est =O, $OW_1$, ou $OCOW_1$;
- $R_2$ est H, un groupe alkyle en $C_1$ à $C_6$, alcényle en $C_2$ à $C_6$, alcynyle en $C_2$ à $C_6$ ou aryl-alkyle en $C_1$ à $C_6$;
- $R_3$ est un groupe alcényle en $C_2$ à $C_6$, alcynyle $C_2$ à $C_6$, arylalkyle en $C_1$ à $C_6$, $CR_4=CR_4C(R_4)_2Z$, $CR_4=CR_4C(R_4)_2OR_5$, $CR_4=CR_4C(R_4)_2OR_4$;
- $R_4$ est H, un groupe alkyle en $C_1$ à $C_6$, alcényle en $C_2$ à $C_6$, aryle, aryl-alkyle $C_1$ à $C_6$ ou alcynyle en $C_2$ à $C_6$;
- $R_5$ est $COW_3$, $CSW_3$ ou $C(NR_4)W_3$;
- X et Y sont, indépendamment, H, un groupe alkyle en $C_1$ à $C_6$, Z, $OR_4$, $OR_5$, $SR_4$, $SR_5$, $N(R_4)_2$, $N(R_5)_2$, $NR_4R_5$, $NR_4OR_4$ ou $NR_4OR_5$; ou X et Y, pris ensemble, sont $NR_4$, $NR_5$, $NOR_4$, $NOR_5$, S, $C(R_4)_2$, $C(R_5)_2$, $CR_5R_4$ ou O;
- Z est un halogène;
- $W_1$ est H, un groupe alkyle en $C_1$ à $C_{20}$, alcényle en $C_2$ à $C_{20}$, aryle, aryl-alkyle en $C_1$ à $C_{20}$ ou alcynyle en $C_2$ à $C_{20}$,
- $W_3$ est H, un groupe alkyle en $C_1$ à $C_6$, alcényle en $C_2$ à $C_6$, aryle, aryl-alkyle en $C_1$ à $C_6$, alcynyle en $C_2$ à $C_6$, $OR_4$ ou $N(R_4)_2$;

et ses sels acceptables du point de vue physiologique, à condition que lorsque X est OH, $OR_5$, $N(R_4)_2$, $N(R_5)_2$, $NR_4R_5$, $SR_5$, $NR_4OR_4$ ou $NR_4OR_5$, Y soit alors autre que Z, OH, $OR_5$, $SR_5$, $NR_4OR_4$, $NR_4OR_5$, $N(R_4)_2$ ou $N(R_5)_2$.

**2.** Composé suivant la revendication 1, dans lequel:

- $R_2$ est H, un groupe alkyle en $C_1$ à $C_6$ ou aryl-alkyle en $C_1$ à $C_6$;
- $R_3$ est un groupe ou alcényle en $C_2$ à $C_6$, alcynyle en $C_2$ à $C_6$, aryl-alkyle en $C_1$ à $C_6$; et
- X et Y sont indépendamment, H, un groupe alkyle en $C_1$ à $C_6$, Z, $OR_4$, $OR_5$, $SR_4$, $SR_5$, $N(R_4)_2$, $N(R_5)_2$, $NR_4R_5$, $NR_4OR_4$ ou $NR_4OR_5$; ou X et Y, pris ensemble, sont $NR_4$, $NR_5$, $NOR_4$, $NOR_5$, S, $C(R_4)_2$, $C(R_5)_2$ ou $CR_5R_4$.

**3.** Composé suivant la revendication 1, dans lequel $R_2$ est un groupe alcényle en $C_2$ à $C_6$ ou alcynyle en $C_2$ à $C_6$.

**4.** Composé suivant la revendication 1, dans lequel $R_3$ est $CR_4=CR_4C(R_4)_2Z$, $CR_4=CR_4C(R_4)_2OR_5$, $CR_4=CR_4C(R_4)_2OR_4$.

**5.** Composé suivant la revendication 1, dans lequel X et Y, pris ensemble, sont un atome d'oxygène.

**6.** Composé suivant la revendication 1, dans lequel $R_3$ est un groupe alcényle en $C_2$ à $C_6$, aryl-alkyle en $C_1$ à $C_6$ ou alcynyle en $C_2$ à $C_6$.

**7.** Composé suivant la revendication 1, dans lequel $R_3$ est un groupe alcényle en $C_2$ à $C_6$ ou aryl-alkyle en $C_1$ à $C_6$.

**8.** Composé suivant la revendication 1, dans lequel X et Y sont, indépendamment, H, un groupe alkyle en $C_1$ à $C_6$, $OR_4$, $OR_5$, $SR_5$, $SR_4$, $N(R_4)_2$, $N(R_5)_2$, $NR_4R_5$, $NR_4OR_4$ ou $NR_4OR_5$; ou X et Y, pris ensemble, sont $NR_4$, $NR_5$, $NOR_4$, $NOR_5$, S, $C(R_4)_2$, $C(R_5)_2$ ou $CR_4R_5$.

**9.** Composé suivant la revendication 1, dans lequel X et Y sont, indépendamment, H, un groupe alkyle en $C_1$ à $C_6$, Z, $OR_4$, $OR_5$, $SR_5$, $SR_4$, $N(R_4)_2$, $NR_4OR_4$, $NR_4OR_5$, $N(R_5)_2$ ou $NR_4R_5$; ou X et Y, pris ensemble, sont $NR_4$, $NR_5$, $NOR_5$, S, $C(R_4)_2$, $C(R_5)_2$ ou $CR_4R_5$.

**10.** Composé selon la revendication 1, dans lequel:

$R_1$ est OH;
$R_2$ est H, ou $CH_3$;
$R_3$ est $CH_2CH=CH_2$; et
X et Y, indépendamment, sont H, F, ou OH;
ou X et Y, pris ensemble, sont NOH.

**11.** Composé suivant la revendication 1, dans lequel:

$R_1$ est OH;
$R_2$ est H, ou $CH_3$;
$R_3$ est $CH_2CH=CH_2$; et
X et Y, indépendamment, sont H ou OH.

**12.** Composé suivant la revendication 1, qui est le 4,4-diméthyl-14α-(l'-hydroxy-2-propényl)-5α-cholest-8-èn-3β-ol;
ou la 14α-allyl-4,4-diméthyl-5α-cholest-8-èn-3β-ol-15-oxime.

**13.** Composition convenable pour inhiber l'activité de lanostérol-8,24-dièn-3β-ol 14α-méthyl-déméthylase comprenant:

(i) une quantité efficace d'un composé de formule:

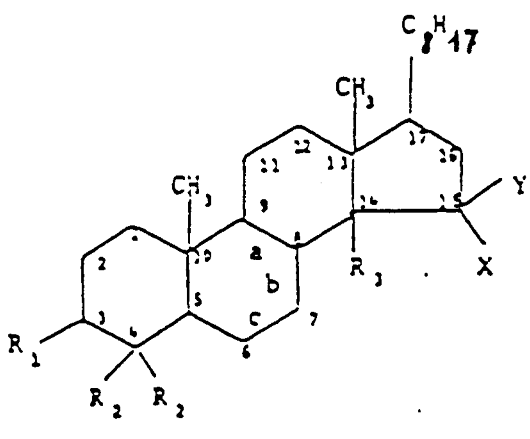

[ I ]

dans laquelle la structure cyclique peut être complètement saturée ou peut être insaturée entre l'une des positions des atomes de carbone 6-7, 7-8 ou 8-9 ou entre les deux positions des atomes de carbone 6-7 et 8-9; et dans laquelle

$R_1$ est =O, $OW_1$, ou $OCOW_1$;
$R_2$ est H, un groupe alkyle en $C_1$ à $C_6$, alcényle en $C_2$ à $C_6$, alcynyle en $C_2$ à $C_6$ ou aryl-

alkyle en $C_1$ à $C_6$;

$R_3$ est un groupe alcényle en $C_2$ à $C_6$, alcynyle $C_2$ à $C_6$, aryl-alkyle en $C_1$ à $C_6$, $CR_4=CR_4C(R_4)_2Z$, $CR_4=CR_4C(R_4)_2OR_5$, $CR_4=CR_4C(R_4)_2OR_4$;

$R_4$ est H, un groupe alkyle en $C_1$ à $C_6$, alcényle en $C_2$ à $C_6$, aryle, aryl-alkyle $C_1$ à $C_6$ ou alcynyle en $C_2$ à $C_6$;

$R_5$ est $COW_3$, $CSW_3$ ou $C(NR_4)W_3$;

X et Y sont, indépendamment, H, un groupe alkyle en $C_1$ à $C_6$, Z, $OR_4$, $OR_5$, $SR_4$, $SR_5$, $N(R_4)_2$, $N(R_5)_2$, $NR_4R_5$, $NR_4OR_4$ ou $NR_4OR_5$; ou

X et Y, pris ensemble, sont $NR_4$, $NR_5$, $NOR_4$, $NOR_5$, S, $C(R_4)_2$, $C(R_5)_2$, $CR_5R_4$ ou O;

Z est un halogène;

$W_1$ est H, un groupe alkyle en $C_1$ à $C_{20}$, alcényle en $C_2$ à $C_{20}$, aryle, aryl-alkyle en $C_1$ à $C_{20}$ ou alcynyle en $C_2$ à $C_{20}$,

$W_3$ est H, un groupe alkyle en $C_1$ à $C_6$, alcényle en $C_2$ à $C_6$, aryle, aryl-alkyle en $C_1$ à $C_6$, alcynyle en $C_2$ à $C_6$, $OR_4$ ou $N(R_4)_2$;

et de ses sels acceptables du point de vue physiologique, à condition que lorsque X est OH, $OR_5$, $N(R_4)_2$, $N(R_5)_2$, $NR_4R_5$, $SR_5$, $NR_4OR_4$ ou $NR_4OR_5$, Y soit alors autre que Z, OH, $OR_5$, $SR_5$, $NR_4OR_4$, $NR_4OR_5$, $N(R_4)_2$ ou $N(R_5)_2$

et

(ii) un support ou un diluant acceptable du point de vue pharmaceutique.

**14.** Composition convenable pour inhiber l'activité de la 3-hydroxy-3-méthylglutaryl coenzyme A réductase comprenant:

(i) une quantité efficace d'un composé de formule:

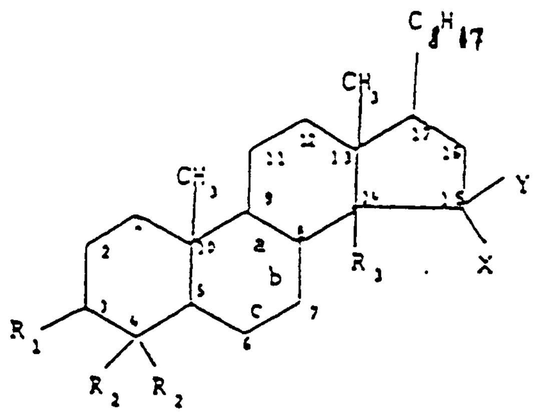

[ I ]

dans laquelle la structure cyclique peut être complètement saturée ou peut être insaturée entre l'une des positions des atomes de carbone 6-7, 7-8 ou 8-9 ou entre les deux positions des atomes de carbone 6-7 et 8-9;

et dans laquelle:

$R_1$ est =O, $OW_1$, ou $OCOW_1$;

$R_2$ est H, un groupe alkyle en $C_1$ à $C_6$, alcényle en $C_2$ à $C_6$, alcynyle en $C_2$ à $C_6$ ou aryl-alkyle en $C_1$ à $C_6$;

$R_3$ est un groupe alcényle en $C_2$ à $C_6$, alcynyle en $C_2$ à $C_6$, aryl-alkyle en $C_1$ à $C_6$, $CR_4=CR_4C(R_4)_2Z$, $CR_4=CR_4$-$C(R_4)_2OR_5$, $CR_4=CR_4C(R_4)_2OR_4$;

$R_4$ est H, un groupe alkyle en $C_1$ à $C_6$, alcényle en $C_2$ à $C_6$, aryle, aryl-alkyle $C_1$ à $C_6$ ou alcynyle en $C_2$ à $C_6$;

$R_5$ est $COW_3$, $CSW_3$ ou $C(NR_4)W_3$;

X et Y sont, indépendamment, H, un groupe alkyle en $C_1$ à $C_6$, Z, $OR_4$, $OR_5$, $SR_4$, $SR_5$, $N(R_4)_2$, $N(R_5)_2$, $NR_4R_5$, $NR_4OR_4$ ou $NR_4OR_5$; ou

X et Y, pris ensemble, sont $NR_4$, $NR_5$, $NOR_4$, $NOR_5$, S, $C(R_4)_2$, $C(R_5)_2$, $CR_5R_4$ ou O;

Z est un atome halogène;

$W_1$ est H, un groupe alkyle en $C_1$ à $C_{20}$, alcényle en $C_2$ à $C_{20}$, aryle, aryl-alkyle en $C_1$ à

$C_{20}$ ou alcynyle en $C_2$ à $C_{20}$,

$W_3$ est H, un groupe alkyle en $C_1$ à $C_6$, alcényle en $C_2$ à $C_6$, aryle, aryl-alkyle en $C_1$ à $C_6$, alcynyle en $C_2$ à $C_6$, $OR_4$ ou $N(R_4)_2$;

et de sels acceptables du point de vue physiologique, à condition que lorsque X est OH, $OR_5$, $N(R_4)_2$, $N(R_5)_2$, $NR_4R_5$, $SR_5$, $NR_4OR_4$ ou $NR_4OR_5$, Y soit alors autre que Z, OH, $OR_5$, $SR_5$, $NR_4OR_4$, $NR_4OR_5$, $N(R_4)_2$ ou $N(R_5)_2$;

et

(ii) un support ou un diluant acceptable du point de vue pharmaceutique.

**15.** Composition convenable pour diminuer la formation du cholestérol comprenant:

(i) une quantité efficace d'un composé de formule:

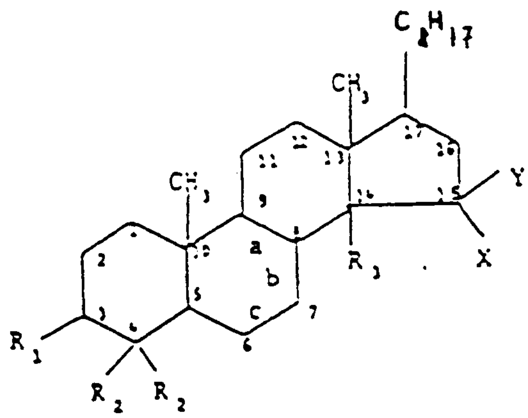

[ I ]

dans laquelle la structure cyclique peut être complètement saturée ou peut être insaturée entre l'une des positions des atomes de carbone 6-7, 7-8 ou 8-9 ou entre les deux positions des atomes de carbone 6-7 et 8-9;

et dans laquelle:

$R_1$ est =O, $OW_1$, ou $OCOW_1$;

$R_2$ est H, un groupe alkyle en $C_1$ à $C_6$, alcényle en $C_2$ à $C_6$, alcynyle en $C_2$ à $C_6$ ou aryl-alkyle en $C_1$ à $C_6$;

$R_3$ est un groupe alcényle en $C_2$ à $C_6$, alcynyle $C_2$ à $C_6$, aryl-alkyle en $C_1$ à $C_6$, $CR_4=CR_4C(R_4)_2Z$, $CR_4=CR_4C(R_4)_2OR_5$, $CR_4=CR_4C(R_4)_2OR_4$;

$R_4$ est H, un groupe alkyle en $C_1$ à $C_6$, alcényle en $C_2$ à $C_6$, aryle, aryl-alkyle $C_1$ à $C_6$ ou alcynyle en $C_2$ à $C_6$;

$R_5$ est $COW_3$, $CSW_3$ ou $C(NR_4)W_3$;

X et Y sont, indépendamment, H, un groupe alkyle en $C_1$ à $C_6$, Z, $OR_4$, $OR_5$, $SR_4$, $SR_5$, $N(R_4)_2$, $N(R_5)_2$, $NR_4R_5$, $NR_4OR_4$ ou $NR_4OR_5$; ou

X et Y, pris ensemble, sont $NR_4$, $NR_5$, $NOR_4$, $NOR_5$, S, $C(R_4)_2$, $C(R_5)_2$, $CR_5R_4$ ou O;

Z est un atome d'halogène;

$W_1$ est H, un groupe alkyle en $C_1$ à $C_{20}$, alcényle en $C_2$ à $C_{20}$, aryle, aryl-alkyle en $C_1$ à $C_{20}$ ou alcynyle en $C_2$ à $C_{20}$,

$W_3$ est H, un groupe alkyle en $C_1$ à $C_6$, alcényle en $C_2$ à $C_6$, aryle, aryl-alkyle en $C_1$ à $C_6$, alcynyle en $C_2$ à $C_6$, $OR_4$ ou $N(R_4)_2$;

et de ses sels acceptables du point de vue physiologique, à condition que lorsque X est OH, $OR_5$, $N(R_4)_2$,$N(R_5)_2$, $NR_4R_5$, $SR_5$, $NR_4OR_4$ ou $NR_4OR_5$, Y soit alors autre que Z, OH, $OR_5$, $SR_5$, $NR_4OR_4$, $NR_4OR_5$, $N(R_4)_2$ ou $N(R_5)_2$;

et

(ii) un support ou un diluant acceptable du point de vue pharmaceutique.

**16.** Composition convenable pour abaisser les taux de cholestérol dans les sérum, comprenant:

(i) une quantité efficace d'un composé de formule:

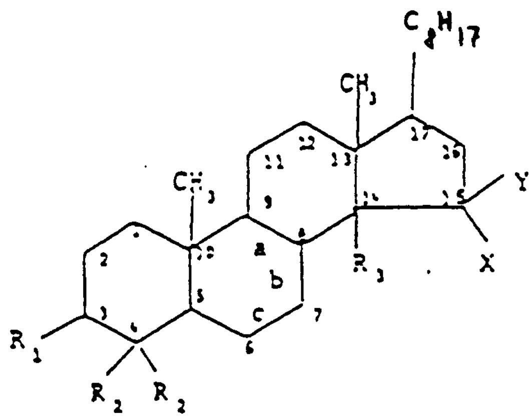

[ I ]

dans laquelle la structure cyclique peut être complètement saturée ou peut être insaturée entre l'une des positions des atomes de carbone 6-7, 7-8 ou 8-9 ou entre les deux positions des atomes de carbone 6-7 et 8-9;
et dans laquelle:

- $R_1$ est =O, $OW_1$, ou $OCOW_1$;
- $R_2$ est H, un groupe alkyle en $C_1$ à $C_6$, alcényle en $C_2$ à $C_6$, alcynyle en $C_2$ à $C_6$ ou aryl-alkyle en $C_1$ à $C_6$;
- $R_3$ est un groupe alcényle en $C_2$ à $C_6$, alcynyle $C_2$ à $C_6$, aryl-alkyle en $C_1$ à $C_6$, $CR_4=CR_4C(R_4)_2Z$, $CR_4=CR_4C(R_4)_2OR_5$, $CR_4=CR_4C(R_4)_2OR_4$;
- $R_4$ est H, un groupe alkyle en $C_1$ à $C_6$, alcényle en $C_2$ à $C_6$, aryle, aryl-alkyle $C_1$ à $C_6$ ou alcynyle en $C_2$ à $C_6$;
- $R_5$ est $COW_3$, $CSW_3$ ou $C(NR_4)W_3$;
- X et Y sont, indépendamment, H, un groupe alkyle en $C_1$ à $C_6$, Z, $OR_4$, $OR_5$, $SR_4$, $SR_5$, $N(R_4)_2$, $N(R_5)_2$, $NR_4R_5$, $NR_4OR_4$ ou $NR_4OR_5$; ou
  X et Y, pris ensemble, sont $NR_4$, $NR_5$, $NOR_4$, $NOR_5$, S, $C(R_4)_2$, $C(R_5)_2$, $CR_5R_4$ ou O;
- Z est un atome d'halogène;
- $W_1$ est H, un groupe alkyle en $C_1$ à $C_{20}$, alcényle en $C_2$ à $C_{20}$, aryle, aryl-alkyle en $C_1$ à $C_{20}$ ou alcynyle en $C_2$ à $C_{20}$,
- $W_3$ est H, un groupe alkyle en $C_1$ à $C_6$, alcényle en $C_2$ à $C_6$, aryle, aryl-alkyle en $C_1$ à $C_6$, alcynyle en $C_2$ à $C_6$, $OR_4$ ou $N(R_4)_2$;

et de ses sels acceptables du point de vue physiologique, à condition que lorsque X est OH, $OR_5$, $N(R_4)_2$,$N(R_5)_2$, $NR_4R_5$, $SR_5$, $NR_4OR_4$ ou $NR_4OR_5$, Y soit alors autre que Z, OH, $OR_5$, $SR_5$, $NR_4OR_4$, $NR_4OR_5$, $N(R_4)_2$ ou $N(R_5)_2$;
et
(ii) un support ou un diluant acceptable du point de vue pharmaceutique.

**Revendications pour l'Etat contractant suivant : ES**

**1.** Procédé pour produire un composé de formule:

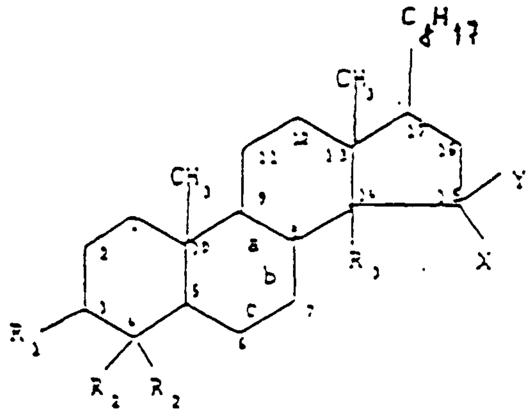

( I )

dans laquelle la structure cyclique peut être complètement saturée ou peut être insaturée entre l'une des positions des atomes de carbone 6-7, 7-8 ou 8-9 ou entre les deux positions des atomes de carbone 6-7 et 8-9,

et dans laquelle:

- $R_1$ est =O, $OW_1$, ou $OCOW_1$;
- $R_2$ est H, un groupe alkyle en $C_1$ à $C_6$, alcényle en $C_2$ à $C_6$, alcynyle en $C_2$ à $C_6$ ou aryl-alkyle en $C_1$ à $C_6$;
- $R_3$ est un groupe alcényle en $C_2$ à $C_6$, alcynyle $C_2$ à $C_6$, arylalkyle en $C_1$ à $C_6$, $CR_4=CR_4C(R_4)_2Z$, $CR_4=CR_4C(R_4)_2OR_5$, $CR_4=CR_4C(R_4)_2OR_4$;
- X et Y sont, indépendamment, H, un groupe alkyle en $C_1$ à $C_6$, Z, $OR_4$, $OR_5$, $SR_4$, $SR_5$, $N(R_4)_2$, $N(R_5)_2$, $NR_4R_5$, $NR_4OR_4$ ou $NR_4OR_5$; ou
  X et Y, pris ensemble, sont $NR_4$, $NR_5$, $NOR_4$, $NOR_5$, S, $C(R_4)_2$, $C(R_5)_2$, $CR_5R_4$ ou O;
- $R_4$ est H, un groupe alkyle en $C_1$ à $C_6$, alcényle en $C_2$ à $C_6$, aryle, aryl-alkyle $C_1$ à $C_6$ ou alcynyle en $C_2$ à $C_6$;
- $R_5$ est $COW_3$, $CSW_3$ ou $C(NR_4)W_3$;
- Z est un atome halogène;
- $W_1$ est H, un groupe alkyle en $C_1$ à $C_{20}$, alcényle en $C_2$ à $C_{20}$, aryle, aryl-alkyle en $C_1$ à $C_{20}$ ou alcynyle en $C_2$ à $C_{20}$,
- $W_3$ est H, un groupe alkyle en $C_1$ à $C_6$, alcényle en $C_2$ à $C_6$, aryle, aryl-alkyle en $C_1$ à $C_6$, alcynyle en $C_2$ à $C_6$, $OR_4$ ou $N(R_4)_2$;

et leurs sels acceptables du point de vue physiologique;

à condition que lorsque X est OH, $OR_5$, $N(R_4)_2$, $N(R_5)_2$, $NR_4R_5$, $SR_5$, $NR_4OR_4$ ou $NR_4OR_5$, Y soit alors autre que Z, OH, $OR_5$, $SR_5$, $NR_4OR_4$, $NR_4OR_5$, $N(R_4)_2$ ou $N(R_5)_2$; dans laquelle $R_3$ est un groupe alkyle en $C_1$ à $C_6$,

comprenant: la réaction d'un composé suivant la revendication 1 dans lequel $R_3$ est H ou n'est pas substitué, avec un agent d'alkylation ou d'alcylation substitué ou non-substitué selon ce qui convient, lorsque cela est approprié, avec un agent oxydant.

**2.** Procédé pour produire un composé suivant la revendication 1, dans lequel:

- $R_3$ est un groupe alcényle en $C_2$ à $C_6$, alcynyle en $C_2$ à $C_6$, $CR_4=CR_4C(R_4)_2Z$, $CR_4=CR_4C(R_4)_2OR_5$, $CR_4=CR_4C(R_4)_2OR_4$,
- $R_4$ est H, un groupe alkyle en $C_1$ à $C_6$, alcényle en $C_2$ à $C_6$, aryle, aryl-alkyle en $C_1$ à $C_6$ ou alcynyle en $C_2$ à $C_6$;

comprenant: la réaction d'un composé suivant la revendication 1, dans lequel $R_3$ est $R_5$, avec un carbanion et, lorsque cela est approprié, avec un agent déshydratant.

**3.** Procédé pour produire un composé suivant la revendication 1, dans lequel X et Y, pris ensemble, sont un atome d'oxygène, comprenant: la réaction d'un composé de formule:

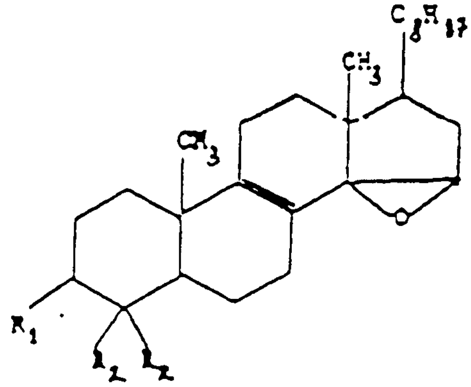

dans laquelle:

$R_1$ est $=O$, $OW_1$, ou $OCOW_1$; et

$R_2$ est H, un groupe alkyle en $C_1$ à $C_6$, alcényle en $C_2$ à $C_6$, alcynyle en $C_2$ à $C_6$ ou aryl-alkyle en $C_1$ à $C_6$,

avec un acide de Lewis et, lorsque cela est approprié, un agent alcylant substitué ou non-substitué.

4. Procédé pour produire un composé suivant la revendication 1, dans lequel X et Y, pris ensemble, sont $NR_4$, $NR_5$, $NOR_4$ ou $NOR_5$, comprenant: la réaction d'un composé suivant la revendication 1 dans lequel X et Y, pris ensemble, sont un atome d'oxygène, avec un sel d'amine substituée ou non substituée dans une base.

5. Procédé pour produire un composé suivant la revendication 1, dans lequel X et Y, pris ensemble, sont un atome de soufre, comprenant: la réaction d'un composé suivant la revendication 1 dans lequel X et Y, pris ensemble, sont un atome d'oxygène, avec un agent de thiolation.

6. Procédé pour produire un composé suivant la revendication 1, dans lequel X et Y, sont indépendamment un groupe alkyle en $C_1$ à $C_6$ ou X et Y, pris ensemble, sont $C(R_4)_2$, $C(R_5)_2$, $CR_5R_4$, comprenant: la réaction d'un composé suivant la revendication 1 dans lequel X et Y, pris ensemble, sont un atome d'oxygène, avec un carbanion et, lorsque cela est approprié, un agent déshydratant.

7. Procédé pour produire un composé suivant la revendication 1, dans lequel X et Y, indépendamment, sont H, $OR_4$, $OR_5$, $SR_4$, $SR_5$, $N(R_4)_2$, $N(R_5)_2$, $NR_4R_2$, $NR_4OR_4$, $NR_4OR_5$, comprenant: la réaction d'un composé suivant la revendication 1 dans lequel X et Y, pris ensemble, sont $NR_4$, $NR_5$, $NOR_4$, $NOR_5$, S ou O avec un agent réducteur.

8. Procédé pour produire un composé suivant la revendication 1, dans lequel X et Y, indépendamment, sont Z, comprenant: la réaction d'un composé suivant la revendication 1 dans lequel X et Y, sont indépendamment $OR_4$ ou $OR_5$ avec un agent halogénant.